# EUROPEAN PATENT SPECIFICATION

(11) **EP 1 203 813 B1**
(45) Date of publication and mention of the grant of the patent: **25.01.2006**
(21) Application number: 02003408.8
(22) Date of filing: 26.05.1999
(51) Int. Cl.: A61K 35/76, C12N 7/04, A61K 48/00, C12Q 1/70

(54) **Attenuated pestiviruses**
Attenuierte Pestiviren
Pestivirus atténués

(30) Priority: 05.06.1998 EP 98110356
(43) Date of publication of application: 08.05.2002
(62) Divisional of application: 99926430.2
(73) Proprietor: BOEHRINGER INGELHEIM VETMEDICA GMBH, 55216 Ingelheim am Rhein (DE)
(72) Inventor: Meyers, Gregor, 72141 Walddorfhaeslach (DE)
(74) Representative: Kläs, Heinz-Gerd

(56) References cited:
- EP-A- 0 794 257
- M.M. HULST ET AL.: "Inactivation of the RNase activity of Glycoprotein Erns of Classical Swine Fever Virus results in a cytopathogenic virus" JOURNAL OF VIROLOGY., vol. 72, no. 1, January 1998 (1998-01), pages 151-157, XP002095266 ICAN SOCIETY FOR MICROBIOLOGY US
- WINDISCH J M ET AL: "RNase of classical swine fever virus: biochemical characterization and inhibition by virus-neutralizing monoclonal antibodies." JOURNAL OF VIROLOGY, (1996 JAN) 70 (1) 352-8. JOURNAL CODE: KCV. ISSN: 0022-538X., XP002095267 United States

## Description

### Field of the invention

The present invention relates to a method for attenuating pestiviruses by inactivating the ribonuclease activity (RNase activity) residing in glycoprotein E^{RNS}. The invention also relates to pestiviruses attenuated according to the invention, nucleic acids for preparing such pestiviruses, vaccines and pharmaceutical compositions comprising the attenuated pestiviruses of the invention. The invention further relates to methods for distinguishing between the attenuated viruses of the invention and pathogenic viruses.

### Background of the invention

Pestiviruses are causative agents of economically important diseases of animals in many countries worldwide. Presently known virus isolates have been grouped into three different species which together form one genus within the family Flaviviridae.
I Bovine viral diarrhea virus (BVDV) causes bovine viral diarrhea (BVD) and mucosal disease (MD) in cattle (Baker, 1987; Moennig and Plagemann, 1992; Thiel et al., 1996).
II Classical swine fever virus (CSFV), formerly named hog cholera virus, is responsible for classical swine fever (CSF) or hog cholera (HC) (Moennig and Plagemann, 1992; Thiel et al., 1996).
III Border disease virus (BDV) is typically found in sheep and causes border disease (BD). Symptoms similar to MD in cattle have also been described to occur after intrauterine infection of lambs with BDV (Moennig and Plagemann, 1992; Thiel et al., 1996).

An alternative classification of pestiviruses is provided by Becher et al. (1995) or others.

Pestiviruses are small enveloped viruses with a single stranded RNA genome of positive polarity lacking both 5' cap and 3' poly(A) sequences. The viral genome codes for a polyprotein of about 4000 amino acids giving rise to final cleavage products by co- and posttranslational processing involving cellular and viral proteases. The viral proteins are arranged in the polyprotein in the order NH₂-N^{pro}-C-E^{RNS}-E1-E2-p7-NS2-NS3-NS4A-NS4B-NS5A-NS5B-COOH (Rice, 1996). Protein C and the glycoproteins E^{RNS}, E1 and E2 represent structural components of the pestivirus virion (Thiel et al., 1991). E2 and to a lesser extent E^{RNS} were found to be targets for antibody neutralization (Donis et al., 1988; Paton et al., 1992; van Rijn et al., 1993; Weiland et al., 1990,1992). E^{RNS} lacks a membrane anchor and is secreted in considerable amounts from the infected cells; this protein has been reported to exhibit RNase activity (Hulst et al., 1994; Schneider et al., 1993; Windisch et al., 1996). The function of this enzymatic activity for the viral life cycle is presently unknown. In the case of a CSFV vaccine strain experimental destruction of the RNase by site directed mutagenesis has been reported to result in a cytopathogenic virus that has growth characteristics in cell culture equivalent to wild type virus (Hulst et al., 1998). The enzymatic activity depends on the presence of two stretches of amino acids conserved between the pestivirus E^{RNS} and different known RNases of plant and fungal origin. Both of these conserved sequences contain a histidine residue (Schneider et al., 1993). Exchange of each of these residues against lysine in the E^{RNS} protein of a CSFV vaccine strain resulted in the destruction of RNase activity (Hulst et al., 1998). Introduction of these mutations into the genome of the CSFV vaccine strain did not influence viral viability or growth properties but led to a virus exhibiting a slightly cytopathogenic phenotype (Hulst et al., 1998).

Vaccines comprising attenuated or killed viruses or viral proteins expressed in heterologous expression systems have been generated for CSFV and BVDV and are presently used. The structural basis of the attenuation of these viruses used as life vaccines is not known. This leads to the risk of unpredictable revertants by backmutation or recombination subsequent to vaccination. On the other hand, the efficacy of inactivated vaccines or heterologously expressed viral proteins (subunit vaccines) in the induction of immunity is rather low.

In general, live vaccines with defined mutations as a basis for attenuation would allow to avoid the disadvantages of the present generation of vaccines. Potential targets for attenuating mutations in pestiviruses are not available at present.

A further advantage of said attenuating mutations lies in their molecular uniqueness which allows to use them as distinctive labels for an attenuated pestiviruses and to distinguish them from pestiviruses from the field.

Because of the importance of an effective and safe as well as detectable prophylaxis and treatment of pestiviral infections, there is a strong need for live and specifically attenuated vaccines with a high potential for induction of immunity as well as a defined basis of attenuation which can also be distinguished from pathogenic pestiviruses.

Therefore, the technical problem underlying the present invention is to provide specifically attenuated and detectably labeled pestiviruses for use as live attenuated vaccines with a high efficiency for the induction of immunity which, as a result of this method, can also be distinguished from pathogenic pestiviruses from the field.

### Disclosure of the invention

The solution to the above technical problem is achieved by the description and the embodiments characterized in the claims.

It has surprisingly been found that pestiviruses can be specifically attenuated by the inactivation of the RNase activity residing in glycoprotein E^{RNS}.

The attenuated pestiviruses now provide live vaccines of high immunogenicity.

Therefore, one aspect disclosed herewith provides a live vaccine comprising a pestivirus, wherein the RNase activity residing in glycoprotein E^{RNS} is inactivated.

The term "vaccine" as used herein refers to a pharmaceutical composition comprising at least one immunologically active component that induces an immunological response in an animal and possibly but not necessarily one or more additional components that enhance the immunological activity of said active component. A vaccine may additionally comprise further components typical to pharmaceutical compostions. The immunologically active component of a vaccine may comprise complete live organisms in either its original form or as attenuated organisms in a so called modified live vaccine (MLV) or organisms inactivated by appropriate methods in a so called killed vaccine (KV). In another form the immunologically active component of a vaccine may comprise appropriate elements of said organisms (subunit vaccines) whereby these elements are generated either by destroying the whole organism or the growth cultures of such organisms and subsequent purification steps yielding in the desired structure(s), or by synthetic processes induced by an appropriate manipulation of a suitable system like, but not restricted to bacteria, insects, mammalian or other species plus subsequent isolation and purification procedures or by induction of said synthetic processes in the animal needing a vaccine by direct incorporation of genetic material using suitable pharmaceutical compositions (polynucleotide vaccination). A vaccine may comprise one or simultaneously more than one of the elements described above.

Additional components to enhance the immune response are constituents commonly referred to as adjuvants, like e.g. aluminiumhydroxide, mineral or other oils or ancillary molecules added to the vaccine or generated by the body after the respective induction by such additional components, like but not restricted to interferons, interleukins or growth factors.

A "pharmaceutical composition" essentially consists of one or more ingredients capable of modifying physiological e.g. immunological functions of the organism it is administered to, or of organisms living in or on its surface like but not restricted to antibiotics or antiparasitics, as well as other constituents added to it in order to achieve certain other objectives like, but not limited to, processing traits, sterility, stability, feasibility to administer the composition via enteral or parenteral routes such as oral, intranasal, intravenous, intramuscular, subcutaneous, intradermal or other suitable route, tolerance after administration, controlled release properties.

A vaccine of the invention refers to a vaccine as defined above, wherein one immunologically active component is a pestivirus or of pestiviral origin.

The term "live vaccine" refers to a vaccine comprising a living, in particular, a living viral active component.

The term "pestivirus" as used herein refers to all pestiviruses, characterized by belonging to the same genus as BVDV, CSFV and BDV within the family Flaviviridae and by their expression of glycoprotein E^{RNS}. Of course, said term also refers to all pestiviruses as characterized by Becher et al. (1995) or others that express glycoprotein E^{RNS}. "RNase activity" as used herein refers to the ability of the glycoprotein E^{RNS} to hydrolyze RNA.

It should be noted that the term glycoprotein E0 is often used synonymously to glycoprotein E^{RNS} in publications.

The term "inactivation of the RNase activity residing in said glycoprotein" refers to the inability or reduced capability of a modified glycoprotein E^{RNS} to hydrolyze RNA as compared to the unmodified wild type of said glycoprotein E^{RNS}.

Inactivation of the RNase activity residing in glycoprotein E^{RNS} can be achieved by deletions and/or mutations of at least one amino acid of said glycoprotein as demonstrated herein and by Hulst et al. (1998). Therefore, in a preferred embodiment the present invention relates to live vaccines, wherein said RNase activity is inactivated by deletions and/or mutations of at least one amino acid of said glycoprotein.

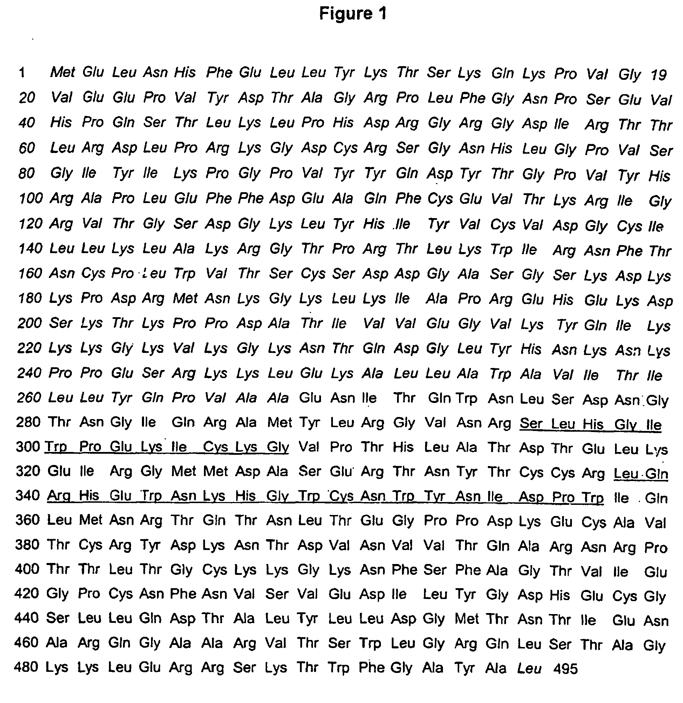

It has been shown that the glycoprotein E^{RNS} forms a disulfide-bonded homodimer of about 97 kD, wherein each monomer consists of 227 amino acids corresponding to the amino acids 268 to 494 of the CSFV polyprotein as described by Rümenapf et al. (1993). The first 495 amino acids as expressed by the Alfort strain of CSFV are shown in figure 1 for reference purpose only. The genome sequence of the Alfort strain of CSFV is available in the GenBank/EMBL data library under accession number J04358; alternatively, the amino acid sequence for the BVDV strain CP7 can be accessed in the GenBank/EMBL data library (accession number U63479). Two regions of amino acids are highly conserved in glycoprotein E^{RNS} as well as in some plant and fungal RNase-active proteins (Schneider et al., 1993). These two regions are of particular importance to the RNase enzymatic activity. The first region consists of the region at the amino acids at position 295 to 307 and the second region consists of the amino acids at position 338 to 357 of said viral polyprotein as exemplified by figure 1 for the Alfort strain of CSFV (numbering according to the published deduced amino acid sequence of CSFV strain Alfort (Meyers et al., 1989). The amino acids of particular importance to the RNase activity as mentioned above are by no means limited to the exact position as defined for the Alfort strain of CSFV but are simply used in an exemplary manner to point out the preferred amino acids being at that position or corresponding to that position in other strains such as found in BVDV, BDV and pestiviruses in general since they are highly conserved. For pestiviruses other than the CSFV Alfort strain the numbering of the positions of the preferred amino acids is often different but an expert in the field of the molecular biology of pestiviruses will easily identify these preferred amino acids by their position relative to the highly conserved amino acids of said glycoprotein. In one particular non-limiting example, the position of CSFV Alfort 346 is identical to position 349 of BVDV strain cp7.

As a consequence, a more preferred embodiment disclosed herewith relates to a vaccine as provided herewith, wherein said inactivating deletions and/or mutations are located at the amino acids at position 295 to 307 and/or position 338 to 357, as described in figure 1 for the CSFV Alfort strain in an exemplary manner or corresponding thereto in other strains, of said glycoprotein.

A very preferred embodiment discloses that the inactivation of said RNase activity by deletion or mutation of the amino acid at position 346 of said glycoprotein leads to particularly useful live vaccines. Therefore, the present invention relates to vaccines provided herewith, wherein said Rnase activity is inactivated by deletion or mutation of the amino acid at position 346, as described in figure 1 for the CSFV Alfort strain in an exemplary manner or corresponding thereto in other strains, of said glycoprotein.

The present disclosure demonstrates that pestiviruses are viable and code for an E^{RNS} protein without RNase activity when the histidine residue at position 346 of the viral polyprotein (numbering according to the published sequence of CSFV Alfort/Tübingen (Meyers et al., 1989)), which represents one of the conserved putative active site residues of the E^{RNS} RNase, is deleted. It has also been demonstrated for this invention that the deletion of the respective histidine in the E^{RNS} of a BVD pestivirus (position 349, numbered according to the sequence of BVDV CP7 GenBank/EMBL data library (accession number U63479)) results in a viable virus in which the E^{RNS} glycoprotein has lost the RNase activity. In contrast to point mutations changing one amino acid into another, a deletion mutant is generally much more stable with respect to revertants. Infection of pigs with a mutant of the pathogenic CSFV Alfort/Tübingen expressing E^{RNS} with this deletion did not lead to fever or other typical clinical signs of CSFV infections whereas the infection with wild type virus resulted in fever, diarrhea, anorexia, apathy, depletion of B-cells and central nervous disorders. These pigs were killed in a moribund stage showing severe hemorrhages in the skin and internal organs 14 days post inoculation. The pigs infected with the mutant did neither show viremia nor B-cell depletion as tested on days 3, 5, 7, 10, 14 post infection while CSFV was easily isolated from blood samples derived from the pigs inoculated with wild type virus. The deletion mutant apparently replicated in the animals as indicated by the induction of neutralizing antibodies (see Example 3, Table 3c). The immune response to the mutant virus was sufficient to permit to survive a lethal challenge with 2x10⁵ TCID₅₀ of the highly pathogenic infection with the CSFV strain Eystrup (König, 1994) which is heterologous to the Alfort strain. Moreover, the tested animals displayed no typical clinical signs for CSFV infection like fever, diarrhea, hemorrhages, B-cell depletion or anorexia after the challenge infection. This data demonstrates that infection of pigs with the deletion mutant induces an immune response sufficient for protection against a stringent challenge.

Therefore, a most preferred embodiment disclosed herewith relates to vaccines according to the invention, wherein said RNase activity is inactivated by the deletion of the histidine residue at position 346, as described in figure 1 for the CSFV Alfort strain in an exemplary manner or corresponding thereto in other strains, of said glycoprotein.

A further most preferred embodiment disclosed herewith relates to BVDV vaccines according to the invention, wherein said RNase activity is inactivated by the deletion of the histidine residue at position 346, as described in figure 1 for the CSFV Alfort strain in an exemplary manner or corresponding thereto in other BVDV strains, of said glycoprotein.

Another aspect disclosed herewith relates to attenuated pestiviruses, wherein the RNase activity residing in glycoprotein E^{RNS} is inactivated by deletions and/or mutations of at least one amino acid of said glycoprotein with the proviso that the amino acids at position 297 and/or 346 of said glycoprotein as described in figure 1 for CSFV are not lysine. A recombinant pestivirus, wherein amino acids at position 297 and/or 346 of said glycoprotein are lysine has been described by Hulst et al. in 1998. These particular pestiviruses demonstrated cytopathic effects in swine kidney cells. Up to now, there has been total unawareness of the surprising and innovative attenuating feature due to the inactivation of the E^{RNS} enzymatic activity.

A preferred embodiment disclosed herewith, for the reasons stated above for vaccines, also relates to pestiviruses as provided herewith, wherein said RNase activity is inactivated by deletions and/or mutations located at the amino acids at position 295 to 307 and/or position 338 to 357, as described in figure 1 for the CSFV Alfort strain in an exemplary manner or corresponding thereto in other strains, of said glycoprotein.

A more preferred embodiment disclosed herewith, for the reasons stated above for vaccines, also relates to pestiviruses as provided herewith, wherein said RNase activity is inactivated by deletion or mutation of the amino acid at position 346, as described in figure 1 for the CSFV Alfort strain in an exemplary manner or corresponding thereto in other strains, of said glycoprotein.

A most preferred embodiment disclosed herewith, for the reasons stated above for vaccines, also relates to pestiviruses, wherein said RNase activity is inactivated by the deletion of the histidine residue at position 346, as described in figure 1 for the CSFV Alfort strain in an exemplary manner or corresponding thereto in other strains, of said glycoprotein.

A further most preferred embodiment disclosed herewith relates to BVDV pestiviruses, wherein said RNase activity is inactivated by the deletion of the histidine residue at position 346, as described in figure 1 for the CSFV Alfort strain in an exemplary manner or corresponding thereto in other BVDV strains, of said glycoprotein.

The attenuated pestiviruses and active components of the vaccines of the present invention can easily be prepared by nucleic acid-modifying recombinant techniques resulting in the expression of a mutant amino acid sequence in glycoprotein E^{RNS}. Therefore, a further aspect disclosed herewith relates to nucleic acids coding for a glycoprotein E^{RNS}, wherein the RNase activity residing in said glycoprotein is inactivated by deletions and/or mutations of at least one amino acid of said glycoprotein with the proviso that the amino acids at position 297 and/or 346 of the glycoprotein as described in figure 1 for the CSFV Alfort strain are not lysine.

A preferred embodiment disclosed herewith relates, for reasons as mentioned above, to nucleic acidsas provided herewith , wherein said RNase activity is inactivated by deletions and/or mutations that are located at the amino acids at position 295 to 307 and/or position 338 to 357, as described in figure 1 for the CSFV Alfort strain in an exemplary manner or corresponding thereto in other strains, of said glycoprotein.

A more preferred embodiment disclosed herewith relates, for reasons as mentioned for vaccines, to nucleic acids as provided herewith, wherein said RNase activity is inactivated by deletion or mutation of the amino acid at position 346, as described in figure 1 for the CSFV Alfort strain in an exemplary manner or corresponding thereto in other strains, of said glycoprotein.

A most preferred embodiment disclosed herewith relates to nucleic acids as provided herewith, wherein said RNase activity is inactivated by the deletion of the histidine residue at position 346, as described in figure 1 for the CSFV Alfort strain in an exemplary manner or corresponding thereto in other strains, of said glycoprotein.

A further most preferred embodiment disclosed herewith relates to BVDV nucleic acids according to the invention, wherein said RNase activity is inactivated by the deletion of the histidine residue at position 346, as described in figure 1 for the CSFV Alfort strain in an exemplary manner or corresponding thereto in other BVDV strains, of said glycoprotein.

Nucleotides, e.g. DNA or RNA, are also useful for preparing DNA-, RNA- and/or vector-vaccines. In these vaccines, the nucleotides are applied directly to the animal or indirectly via vectors other than the original virus. Nucleotide vaccines and vector vaccines are well known from the present state of the art and will not be elaborated further.

A further aspect disclosed herewith relates to the use of nucleic acids of the present invention for preparing nucleotide- and/or vector-vaccines.

The vaccines, attenuated pestiviruses, and/or nucleic acids disclosed herewith are particularly useful for the preparation of a pharmaceutical composition.

In consequence, a further aspect disclosed herewith relates to pharmaceutical compositions comprising a vaccine as provided herewith , and/or a pestivirus as provided herewith , and/or a nucleotide sequence as provided herewith.

One non-limiting example of such a pharmaceutical composition, solely given for demonstration purposes, could be prepared as follows: Cell culture supernatant of an infected cell culture is mixed with a stabilizer (e.g. spermidine and/or BSA (bovine serum albumin)) and the mixture is subsequently lyophilized or dehydrated by other methods. Prior to vaccination, said mixture is then rehydrated in aquous (e.g. saline, PBS (phosphate buffered saline)) or non-aquous solutions (e.g. oil emulsion, aluminum-based adjuvant).

An additional aspect disclosed herewith relates to a method of attenuation for pestiviruses. The invention provides a unique and unexpected method for attenuating pestiviruses characterized in that the RNase activity residing in glycoprotein E^{RNS} is inactivated.

The specifically attenuated pestiviruses are especially useful for the preparation of vaccines. Therefore, a further additional aspect disclosed herewith relates to methods for producing a specifically attenuated pestivirus vaccine characterized in that the Rnase activity residing in glycoprotein E^{RNS} is inactivated.

The inactivation of the RNase activity residing in glycoprotein E^{RNS} provides a surprising and new method for detectably labeling pestiviruses. The present invention provides a method for detectably labeling pestiviruses characterized in that the RNase activity residing in glycoprotein E^{RNS} is inactivated. The feature of absence of RNase activity residing in the glycoprotein E^{RNS} of pestiviruses of the invention now enables for detectably labeling these pestiviruses. Labeled and unlabeled pestiviruses or the E^{RNS} secreted from pestivirus infected cells in body fluids can clearly be distinguished by the absence or presence of RNase activity of the glycoproteins E^{RNS} upon isolation and assaying such enzymatic activity.

For pestiviruses inactivated in their RNase activity residing in glycoprotein E^{RNS} by deletion and/or mutation, a number of other techniques can be used. Such pestiviruses can easily be detected because of the structural consequences resulting from such deletions and/or mutations. For example, the sequence difference of the nucleic acid sequence of altered glycoprotein E^{RNS} is detectable by nucleic acid sequencing techniques or PCR-techniques (polymerase-chain reaction) as demonstrated in example 8; the altered protein sequence can be detected by specific monoclonal antibodies, that do not recognize unaltered proteins. Vice versa, it is also possible to detect the altered and thereby structurally labeled proteins by the absence of binding to specific monoclonal antibodies that recognize unaltered glycoproteins E^{RNS} under the proviso that the presence of pestiviruses can be established otherwise. And, of course, the deletions and/or mutations abrogating the RNase activity in the labeled viruses will result in different immune responses in animals when compared to the responses resulting from unlabeled pestivirus infections.

A preferred embodiment for all aspects referring to methods for attenuating pestiviruses, methods for producing a specifically attenuated pestivirus vaccine and methods for detectably labeling pestiviruses according to the invention are those methods relating to the inactivation of the glycoprotein E^{RNS}, wherein said RNase activity is inactivated by deletions and/or mutations of at least one amino acid of said glycoprotein.

A more preferred embodiment for all aspects referring to methods for attenuating pestiviruses, methods for producing a specifically attenuated pestivirus vaccine and methods for detectably labeling pestiviruses according to the invention are those methods relating to the inactivation of the glycoprotein E^{RNS}, wherein said deletions and/or mutations are located at the amino acids at position 295 to 307 and/or position 338 to 357, as described in figure 1 for the CSFV Alfort strain in an exemplary manner or corresponding thereto in other strains, of said glycoprotein.

A very preferred embodiment for all aspects referring to methods for attenuating pestiviruses, methods for producing a specifically attenuated pestivirus vaccine and methods for detectably labeling pestiviruses according to the invention are those methods relating to the inactivation of the glycoprotein E^{RNS}, wherein said RNase activity is inactivated by deletion or mutation of the amino acid at position 346, as described in figure 1 for the CSFV Alfort strain in an exemplary manner or corresponding thereto in other strains, of said glycoprotein.

A most preferred embodiment for all aspects referring to methods for attenuating pestiviruses, methods for producing a specifically attenuated pestivirus vaccine and methods for detectably labeling pestiviruses according to the invention are those methods relating to the inactivation of the glycoprotein E^{RNS}, wherein said RNase activity is inactivated by the deletion of the histidine residue at position 346, as described in figure 1 for the CSFV Alfort strain in an exemplary manner or corresponding thereto in other strains, of said glycoprotein.

The present invention discloses vaccines and or other pharmaceutical compositions which are particularly useful for the prophylaxis and treatment of pestivirus infections in animals. Therefore, a further aspect disclosed herewith relates to methods for the prophylaxis and treatment of pestivirus infections in animals characterized in that a vaccine as provided herewith or another pharmaceutical composition as provided herewith is applied to an animal in need of such prophylaxis or treatment.

A further aspect disclosed herewith relates to a process for the preparation of specifically attenuated pestiviruses characterized in that the RNase activity residing in glycoprotein E^{RNS} is inactivated.

Another aspect disclosed herewith relate to a process for the preparation of specifically labeled pestiviruses characterized in that the RNase activity residing in glycoprotein E^{RNS} is inactivated .

A preferred embodiment for all aspects referring to a process for the preparation of specifically attenuated pestiviruses, a process for the preparation of specifically labeled pestiviruses according to the invention are those processes relating to the inactivation of the glycoprotein E^{RNS}, wherein said RNase activity is inactivated by deletions and/or mutations of at least one amino acid of said glycoprotein.

A more preferred embodiment for all aspects referring to a process for the preparation of specifically attenuated pestiviruses, a process for the preparation of specifically labeled pestiviruses according to the invention are those processes relating to the inactivation of the glycoprotein E^{RNS}, wherein said deletions and/or mutations are located at the amino acids at position 295 to 307 and/or position 338 to 357, as described in figure 1 for the CSFV Alfort strain in an exemplary manner or corresponding thereto in other strains, of said glycoprotein.

A very preferred embodiment for all aspects referring to a process for the preparation of specifically attenuated pestiviruses, a process for the preparation of specifically labeled pestiviruses according to the invention are those processes relating to the inactivation of the glycoprotein E^{RNS}, wherein said Rnase activity is inactivated by deletion or mutation of the amino acid at position 346, as described in figure 1 for the CSFV Alfort strain in an exemplary manner or corresponding thereto in other strains, of said glycoprotein.

A most preferred embodiment for all aspects referring to a process for the preparation of specifically attenuated pestiviruses, a process for the preparation of specifically labeled pestiviruses according to the invention are those processes relating to the inactivation of the glycoprotein E^{RNS}, wherein said RNase activity is inactivated by the deletion of the histidine residue at position 346, as described in figure 1 for the CSFV Alfort strain in an exemplary manner or corresponding thereto in other strains, of said glycoprotein.

The vaccines or other pharmaceutical compositions disclosed herewith are useful for the prophylaxis and treatment of pestivirus infections in animals.

Therefore, one aspect disclosed herewith relates to the use of a vaccine as provided herewith for the prophylaxis and treatment of pestivirus infections in animals. In a further aspect disclosed herewith relates to the use of a pharmaceutical composition as provided herewith for the prophylaxis and treatment of pestivirus infections in animals.

Pestiviruses and/or nucleic acids as provided herewith are useful active components of a pharmaceutical composition or a vaccine. Therefore, a further aspect disclosed herewith relates to the use of a pestivirus of the invention and/or a nucleic acid as provided herewith for the preparation of a vaccine or a pharmaceutical composition.

As mentioned above the inactivation of the RNase activity residing in glycoprotein E^{RNS} provides a surprising and new method for labeling pestiviruses.

As a consequence one aspect disclosed herewith relates to methods for distinguishing the detectably labeled pestiviruses according to the invention from unlabeled and possibly pathogenic pestiviruses. Such methods are especially useful for tracing the efficacy of labeled pestiviruses in animals. A vaccine treated animal will prove label-positive after obtaining a sample of such animal and assaying for said label. Unlabeled animals and especially unlabeled animals that prove pestivirus positive can be immediately separated, isolated or slaughtered to remove the imminent danger of spreading the pathogenic infection to other animals.

The present invention provides a method for detectably labeling pestiviruses characterized in that the RNase activity residing in glycoprotein E^{RNS} is inactivated. This feature of absence of RNase activity residing in the glycoprotein E^{RNS} of pestiviruses as provided herewith now enables for detectably labeling these pestiviruses. As a result labeled and unlabeled pestiviruses can clearly be distinguished by the absence or presence of RNase activity of the glycoprotein E^{RNS} upon isolation and assaying such enzymatic activity. The determination of presence or absence of this enzymatic activity upon obtaining a sample containing a pestivirus of interest or material thereof can be performed according to standard methods as, for example, described in Example 2 or in Hulst et al. (1994).

Therefore, a preferred embodiment disclosed herewith relates to a method for distinguishing pestivirus-infected animals from animals vaccinated with a specifically attenuated pestivirus as described herein, comprising the following steps:
(1) Obtaining a sample from an animal of interest suspected of pestivirus infection or a vaccinated animal;
(2) Determining the absence or presence of RNase activity of a glycoprotein E^{RNS} within said sample;
(3) Correlating the absence of RNase activity of glycoprotein E^{RNS} with a vaccinated animal and correlating the presence of said activity with a pestivirus infection of said animal.

The present disclosure provides pestiviruses inactivated in their RNase activity residing in glycoprotein E^{RNS} by deletion and/or mutation. Such pestiviruses are easily detected because of the structural consequences resulting from such deletions and/or mutations. The sequence difference of the E^{RNS} gene coding for the altered glycoprotein E^{RNS} is detectable by sequencing techniques or PCR-techniques. As a result, a preferred embodiment disclosed herewith relates to a method for distinguishing pestivirus-infected animals from animals vaccinated with a specifically attenuated pestivirus as provided herewith, comprising the following steps:
(1) Obtaining a sample from an animal of interest suspected of pestivirus infection or a vaccinated animal;
(2) Identifying the nucleotide sequence of a pestivirus genome or protein within said sample;
(3) Correlating the deletions and/or mutations of the E^{RNS} nucleotide sequence as present in the vaccine with a vaccinated animal and correlating the absence of said deletions and/or mutations with a pestivirus infection of said animal.

Furthermore, the structural changes resulting from the altered protein sequence of the glycoprotein E^{RNS} of pestiviruses as provided herewith can be detected by specific monoclonal or polyclonal antibodies, that do not recognize unaltered proteins. Therefore, in a further embodiment, the present disclosure relates to a method for distinguishing pestivirus-infected animals from animals vaccinated with an attenuated pestivirus as provided herewith , comprising the following steps:
(1) Obtaining a sample from an animal of interest suspected of pestivirus infection or a vaccinated animal;
(2) Identifying a modified E^{RNS} glycoprotein of an attenuated pestivirus by the specific binding of monoclonal or polyclonal antibodies to E^{RNS} glycoproteins present in said sample, said glycoproteins being modified by a method as described herein, whereby said monoclonal or polyclonal antibodies do not bind to unmodified E^{RNS} glycoproteins;
(3) Correlating the specific binding of said monoclonal or polyclonal antibodies with a vaccinated animal and correlating the absence of antibody binding to a pestivirus infection of said animal under the proviso that the presence of pestiviral material in said animal and/or said sample is established otherwise.

Vice versa, it is also possible to detect the altered and thereby structurally labeled proteins by the absence of binding to specific monoclonal or polyclonal antibodies that recognize unaltered glycoproteins E^{RNS} only, if the presence of pestiviruses can be established otherwise. A preferred embodiment disclosed herewith relates to a method for distinguishing pestivirus-infected animals from animals vaccinated with an attenuated pestivirusas provided herewith, comprising the following steps:
(1) Obtaining a sample from an animal of interest suspected of pestivirus infection or a vaccinated animal;
(2) Identifying an unmodified E^{RNS} glycoprotein of a pestivirus by the specific binding of monoclonal or polyclonal antibodies to E^{RNS} glycoproteins present in said sample, said glycoproteins not being modified by a method as provided herewith, whereby said monoclonal or polyclonal antibodies do not bind to modified E^{RNS} glycoproteins;
(3) Correlating the specific binding of said monoclonal or polyclonal antibodies with a pestivirus infection in said animal and correlating the absence of antibody binding to an vaccinated animal under the proviso that the presence of pestiviral material in said animal and/or said sample is established otherwise.

Of course, the structural modification and absence of the RNase activity in the labeled viruses as provided herewith will result in different immune responses in animals when compared to the responses resulting from unlabeled pestivirus infections. The pestiviruses as provided herewith elicit a different and distinct immune response, cellular as well as humoral, that differs from unmodified and possibly pathogenic immune responses. For example, glycoproteins E^{RNS} as provided herewith will result in polyclonal antibodies that are different in their binding specificity when compared to polyclonal antibodies resulting from unmodified glycoproteins. This difference in binding specificity provides a label for distinguishing animals vaccinated with pestiviruses as provided herewith from pestivirus field infected animals. Tests for screening sera for specific polyclonal antibodies that either bind to a wildtype epitope or a marker deletion mutation of that epitope for the purpose of differentiating infected and vaccinated animals have been described, for example for pseudorabies-infected and vaccinated pigs (Kit et al., 1991).

A preferred embodiment disclosed herewith relates to a method for distinguishing pestivirus-infected animals from animals vaccinated with an attenuated pestivirus as provided herewith, comprising the following steps:
(1) Obtaining a sample of polyclonal antibodies from an animal of interest suspected of pestivirus infection or a vaccinated animal;
(2) Identifying any specific binding of said polyclonal antibodies to unmodified glycoprotein E^{RNS} or glycoprotein E^{RNS} as modified according to present disclosure.
(3) Correlating the binding of said polyclonal antibodies to unmodified glycoprotein E^{RNS} with a pestivirus infection and correlating the binding of said polyclonal antibodies to glycoprotein E^{RNS} as modified according to the ipresent disclosure with a vaccinated.

### References

**1. Baker, J.C.** 1987. Bovine viral diarrhea virus: a review. J. Am. Vet. Med.Assoc. **190**: 1449-1458.
**2. Becher, P., König, M., Paton, D.J., Thiel, H.J.,** 1995, Further characterization of border disease virus isolates: evidence for the presence of more than three species within the genus pesivirus. *Virology* 209 (1), 200-206.
**3. Donis, R.O., Corapi, W., and Dubovi, E.J.** 1988. Neutralizing monoclonal antibodies to bovine viral diarrhea virus bind to the 56K to 58K glycoprotein. *J. Gen. Virol.* **69**: 77-86.
**4. Fuerst T.R. et al.** 1986. Eukaryotic transient expression system based on recombinant vaccinia virus that synthesizes bacteriophage T7 RNA polymerase. **Proc. Natl. Acad. Sci.** 83: 8122-8126.
**5. Hulst, M.M., Himes, G., Newbigin, E., Moormann, R.J.M.** 1994. Glycoprotein E2 of classical swine fever virus: expression in insect cells and identification as a ribonuclease. *Virology* **200**: 558-565.
**6. Hulst, M.M., F.E. Panoto, A. Hooekmann, H.G.P. van Gennip., and Moormann, R.J.M.** 1998. Inactivation of the RNase activity of glycoprotein E^{rns} of classical swine fever virus results in a cytopathogenic virus. J. *Virol*. **72**: 151-157.
**7. Kit, M. and S. Kit. 1991.** Sensitive glycoprotein gIII blocking ELISA to distinguish between pseudorabies (Aujeszky's disease) -infected and vaccinated pigs. *Veterinary Microbiology* 28:141-155.
**8. Kunkel, T. A., J. D. Roberts, and R. A. Zakour.** 1987. Rapid and efficient site-specific mutagenesis without phenotypic selection. *Methods Enzymol*. **154**:367-392.
**9. König, Matthias,** 1994, Virus der klassischen Schweinepest: Untersuchungen zur Pathogenese und zur Induktion einer protektiven Immunantwort. Dissertation, Tierärztliche Hochschule Hannover, Germany.
**10. Meyers, G., Rümenapf, T. and Thiel, H.-J.** 1989. Molecular cloning and nucleotide sequence of the genome of hog cholera virus.Virology **171**: 555-567.
**11. Meyers, G., Tautz, N., Becher, P., Thiel, H.-J., & Kümmerer, B.M.** 1996b. Recovery of cytopathogenic and noncytopathogenic bovine viral diarrhea viruses from cDNA constructs. *J. Virol*., **70:** 8606-8613.
**12. Meyers, G., Thiel, H.-J., and Rümenapf, T.** 1996a. Classical swine fever virus: Recovery of infectious viruses from cDNA constructs and generation of recombinant cytopathogenic swine fever virus. *J. Virol*. **67:**7088-709526.
**13. Moennig, V. and Plagemann, J.** 1992. The pestiviruses. Adv. Virus Res. **41:** 53-91.
**14. Paton, D.J., Lowings, J.P., Barrett, A.D.** 1992. Epitope mapping of the gp53 envelope protein of bovine viral diarrhea virus. *Virology* **190:** 763-772.
**15. Pellerin, C. et. al.** Identification of a new group of bovine viral diarrhea virus strains associated with severe outbreaks and high mortalities, Virology **203**, 1994:260-268.
**16.** Rice, C.M. 1996.The pestiviruses. In *Fields Virology,* eds. Fields, B.N., Knipe, D.M., & Howley, P.M. (Lippincott-Raven, Philadelphia), pp. 931-959.
**17. Rümenapf, T., Unger, G., Strauss, J.H., and Thiel, H.-J.** 1993. Processing of the evelope glycoproteins of pestiviruses. J. Virol. **67**: 3288-3294
**18. Schneider, R., G. Unger, R. Stark, E. Schneider-Scherzer, and H.-J. Thiel.** 1993. Identification of a structural glycoprotein of an RNA virus as a ribonuclease. *Science* **261**: 1169-1171.
**19. Thiel, H.-J., Plagemann, G.W., & Moennig, V.** 1996. The pestiviruses. In *Fields Virology,* eds. Fields, B.N., Knipe, D.M., & Howley, P.M. (Lippincott-Raven, Philadelphia), pp.1059-1073.
**20. Thiel, H.-J., Stark, R., Weiland, E., Rümenapf, T. & Meyers, G.** 1991. Hog cholera virus: molecular composition of virions from a pestivirus. *J. Virol.* **65:** 4705-4712.31.
**21. van Rijn, P.A., van Gennip, H.G., de Meijer, E.J., Moormann, R.J.** 1993. Epitope mapping of envelope glycoprotein E1 of hog cholera virus strain Brescia. *J. Gen. Virol.* **74:** 2053-2060.
**22. Weiland, E., Thiel, H.-J., Hess, G., and Weiland, F.** (1989). Development of monoclonal neutralizing antibodies agaist bovine viral diarrhea virus after pretreatment of mice with normal bovine cells and cyclophosphamide. J. Virol. Methods **24**: 237-244.
**23. Weiland, E., Stark, R., Haas, B., Rümenapf, T., Meyers, G. and Thiel, H.-J.** (1990). Pestivirus glycoprotein which induces neutralizing antibodies forms part of a disulfide-linked heterodimer. J. Virology **64**, 3563-3569.
**24. Weiland, E., Ahl, R., Stark, R., Weiland, F. and Thiel, H.-J.** (1992). A second envelope glycoprotein mediates neutralization of a pestivirus, hog cholera virus. J. Virology **66**, 3677-3682.
**25. Windisch, J.M., Schneider, R., Stark, R., Weiland, E., Meyers, G., and Thiel, H.-**J. 1996. RNase of classical swine fever virus: biochemical characterization and inhibition by virus-neutralizing monoclonal antibodies. *J.* V*irol.* **70**: 352-358

### Examples

### Example 1 Generation of RNase-negative pestivirus mutants

Starting with the full length cDNA clones pA/CSFV (Meyers et al., 1996a) or pA/BVDV (Meyers et al., 1996b), from which infectious cRNA can be obtained by in vitro transcription, subclones were generated. For CSFV, a Xhol/Sspl fragment of pA/CSFV was cloned into pBluescript SK+, cut with Xhol and Smal. For BVDV, a XhoI/BgIII fragment from pA/BVDV was cloned into plasmid pCITE-2C, cut with the same enzymes. Single stranded plasmid DNA was produced from these constructs according to the method of Kunkel (Kunkel et al., 1987) using E. coli CJ 236 cells (BioRad) and the VCMS single strand phage (Stratagene). The single stranded DNA was converted to double strands using the 'Phagemid in vitro Mutagenesis Kit' (BioRad). Some of the synthetic oligonucleotides which were used as primers for generating the desired pestivirus mutants are listed below in an exemplary fashion:

The double stranded plasmid DNA was used for transformation of E.coli XL1-Blue cells (Stratagene). Bacterial colonies harboring plasmids were isolated via ampicillin selection. Plasmid DNA was prepared and further analyzed by nucleotide sequencing using the T7 polymerase sequencing kit (Pharmacia). Plasmids containing the desired mutations and no second site changes were used for the construction of full length cDNA clones. In the case of CSFV, a XhoI/NdeI fragment from the mutagenized plasmid was inserted together with a NdeI/BgIII fragment derived from plasmid 578 (pCITE 2A, containing the XhoI/BgIII fragment form pA/CSFV) into pA/CSFV cut with XhoI and BgIII. To obtain the BVDV CP7 mutant, a XhoI/BgIII fragment containing the deletion was inserted into pA/BVDV cut with Xhol and Ncol together with a BgIII/NcoI fragment isolated from pA/BVDV/Ins-. From construct pA/BVDV/Ins- a cRNA was transcribed that gives rise to a noncytopathogenic BVDV upon transfection in suitable cells (Meyers et al., 1996b). The different full length clones were amplified, and the plasmids isolated. The presence of the desired mutations was proven by DNA sequencing. After linearization with Srfl (CSFV full length clones) or Smal (BVDV full length clones) cRNA was transcribed as described previously (Meyers et al., 1996ab). RNA was purified by gel filtration and phenol/chloroform extraction and used for transfection of porcine kidney (PK15) cells or bovine kidney (MDBK clone B2) cells (CSFV or BVDV constructs, respectively). The transfections were analyzed by immunofluorescence with virus specific antisera. In cases where the desired mutants could be recovered (immunofluorescence positive) the viruses were amplified by passage on the same cell lines used for the transfection experiments. Further analysis of the CSFV mutants included determination of one step growth curves and characterization of viral RNA by Northern blot with virus specific cDNA probes as well as reverse transcription polymerase chain reaction (RT-PCR) and subsequent sequencing of the PCR fragments to verify the presence of the desired mutations in the viral genome. In all cases the presence of the desired mutation was proven. All of the recovered viruses grew equally well and produced similar amounts of RNA just as the virus resulting from the plasmid displaying the wild type sequence.

The viability of the BVDV mutant was shown by transfection of the respective cRNA and splitting of the cells 3 days thereafter. Part of the cells was seeded into a 3.5 cm diameter dish, fixed with acetone/methanol at the day thereafter and analyzed by immunofluorescence with a mixture of BVDV-specific monoclonal antibodies (Weiland et al., 1989). All cells were found positive whereas a control of cells transfected with noninfectious RNA showed no signal. From a part of the cells transfected with the respective cRNA, an extract was produced by one cycle of freezing and thawing. Fresh cells were infected with this cell extract and proved to be BVDV positive by BVDV specific immunofluorescence 3 days post infection.

Table 1 summarizes the different changes introduced into the conserved sequences of E^{RNS} representing the putative active site of the RNase which are encoded by the indicated virus mutants

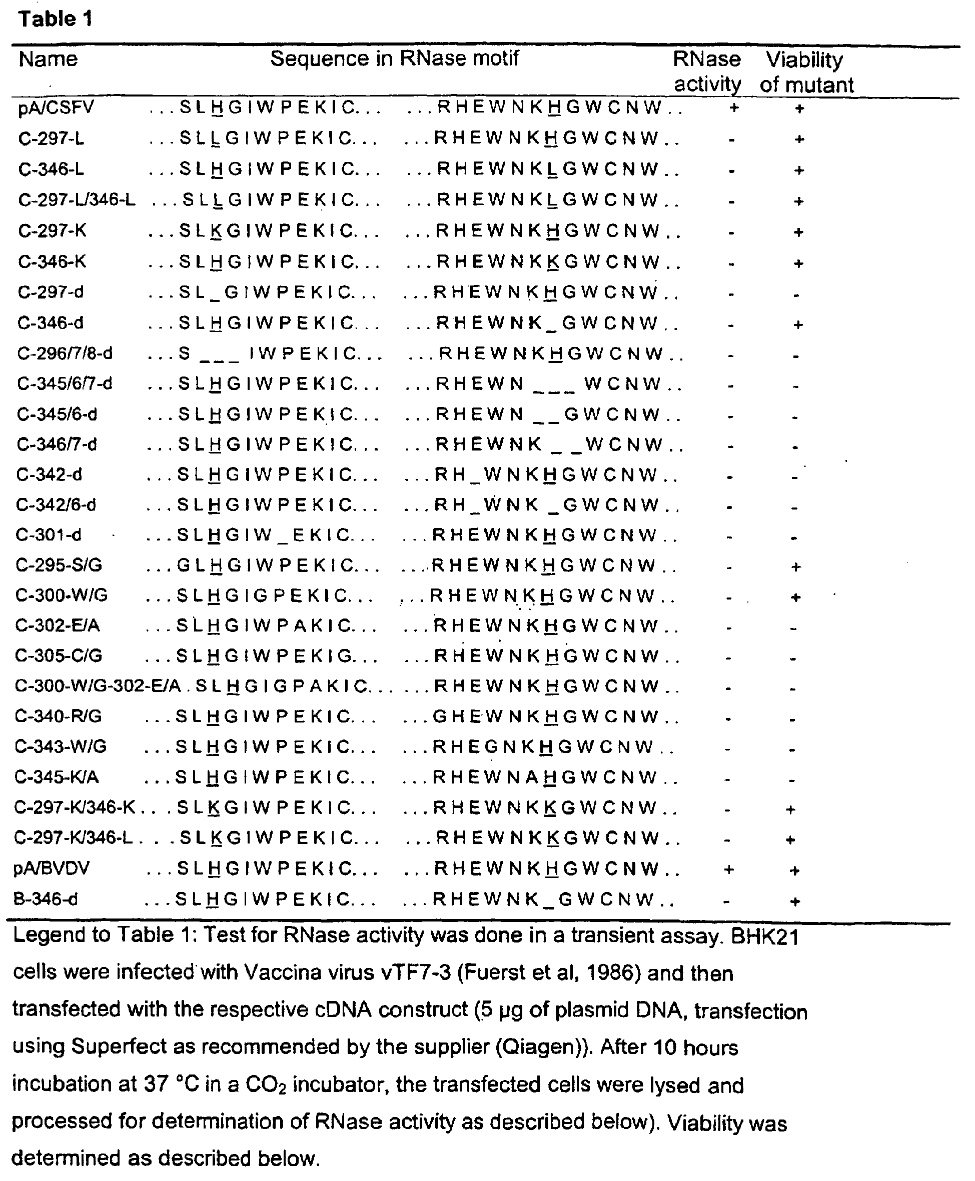

### Example 2 Effect of different mutations on RNase activity of E^{RNS}

To test the effect of the different mutations on the RNase activity of E^{RNS} appropriate cells were infected with the mutant viruses. For CSFV, the infection was carried out with a multiplicity of infection (m.o.i.) of 0.01. Infection with wild type virus served as a positive control whereas noninfected cells were used as a negative control. At 48h post infection, cells were washed twice with phosphate buffered saline and lysed in 0.4 ml of lysis buffer (20 mM Tris/HCl; 100 mM NaCl, 1 mM EDTA, 2 mg/ml bovine serum albumin; 1% Triton X100; 0.1% deoxycholic acid; 0.1% sodium dodecyl sulfate). The lysate was given into 1.5 ml reaction tubes, sonified (Branson sonifier B12, 120 Watt, 20 s in a cup horn water bath), cleared by centrifugation (5 min, 14,000 rpm, Eppendorf Centrifuge, 4°C) and the supernatant subjected to ultracentrifugation (Beckmann table top ultracentifuge, 60 min at 4°C and 45,000 rpm in a TLA 45 rotor). Determination of RNase activity was done in a total volume of 200 µl containing 5 or 50 µl of supernatant of the second centrifugation step and 80 µg of Poly(rU)(Pharmacia) in RNase-assay buffer (40 mM Tris-acetate (pH 6.5), 0.5 mM EDTA, 5 mM dithiothreitol (DTT)). After incubation of the reaction mixture at 37°C for 1 hour 200 µl of 1.2 M perchloric acid, 20 mM LaSO₄ was added. After 15 min incubation on ice the mixture was centrifugated for 15 min at 4°C and 14,000 rpm in an Eppendorf centrifuge. To the supernatant 3 volumes of water were added and an aliquot of the mixture was analyzed by measuring the optical density at 260 nm using an Ultrospec 3000 spectrophotometer (Pharmacia). In all cases, the mutations introduced into the E^{rns} gene completely abrogated RNase activity (Table 1).

For the BVDV mutant RNase activity was tested with material obtained after RNA transfection without passage of the recovered viruses. Cells transfected with the appropriate RNA were split 72h post transfection and seeded in two dishes. 24h later, from one dish, cell extracts were prepared and analyzed for RNase activity as described above. To prove infection, the cells of the second dish were analyzed by immunofluorescence with BVDV specific monoclonal antibodies (Weiland et al., 1989) and found 100% positive. Transfection was carried out with RNA transcribed from pA/BVDV/Ins- and from pA/B-346-d, the plasmid equivalent to pA/BVDV/Ins-but containing the deletion of the codon equivalent to the codon 346 in the CSFV Alfort genome. Nontransfected MDBK cells served as a negative control.

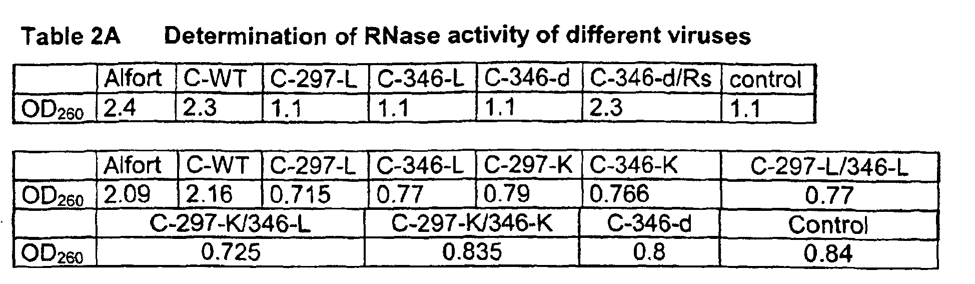

### Description of Table 2A:

PK15 cells were infected with the indicated viruses at an m.o.i. (multiplicity of infection) of 0.01, incubated at 37°C for 48 h in a CO₂ incubator, and then lysed and subjected to RNase test. The acid soluble RNA resulting from incubation with the different cell extracts was quantified by measuring the optical density at 260 nm. The observed differences in RNase activity were not due to different amounts of E^{RNS} protein in the samples since similar values were obtained after quantification of E^{RNS} by radioactive labeling, immunoprecipitation and analysis of radioactivity with a phosphorimager. Moreover, reduction of the E^{rns} concentration in the assay down to only one tenth of the usual amount did not change the resulting OD values considerably, indicating that with the chosen conditions the assay was saturated with E^{ms}.

CSFV strain Alfort; all other viruses were recovered from RNA transcribed in vitro from plasmids: e.g. C-WT from pA/CSFV; C-297-L from pA/C-297-L; etc.; C-346-d/Rs virus was recovered from pA/C-346-d/Rs (generated by reversion of mutation in pA/C-346-d by exchange of the respective cDNA fragment against the equivalent fragment derived from pA/CSFV); control: extract of non-infected PK15 cells.

**Table 2B**

| | B-WT | B-346-d | control |
|---|---|---|---|
| OD₂₆₀ | 2.5 | 1.1 | 1.1 |

### Description of table 2B

MDBK cells were infected with in vitro transcribed RNA, split 72h post transfection and analyzed 24h later for RNase activity. Infection of the cells was proven by immunofluorescence analysis as described in the text.

B-WT: virus recovered from pA/BVDV/Ins-; B-346-d: virus recovered from pA/B-346-d; control; extract from noninfected MDBK cells.

### Example 3 Pathogenicity of CSFV after RNase inactivation

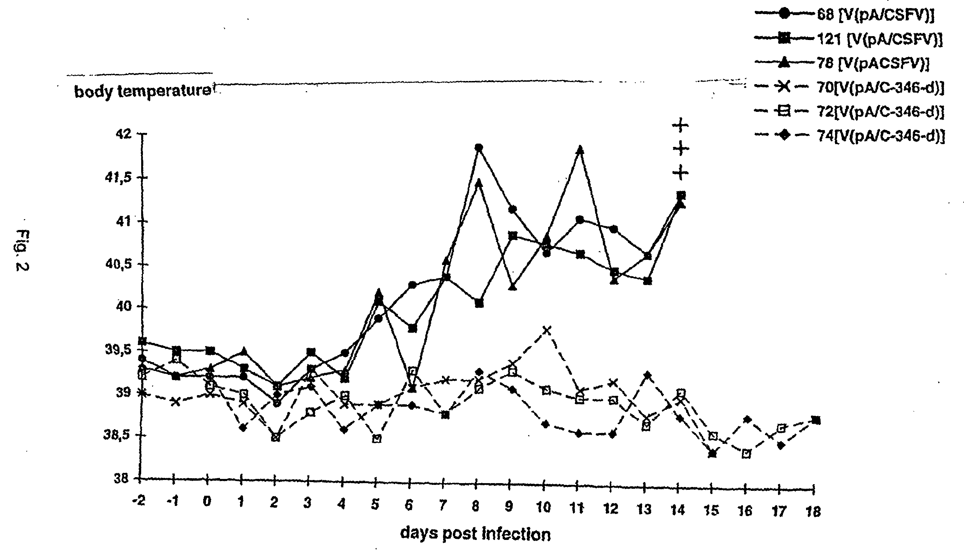

To test, whether the destruction of the RNase activity influences the pathogenicity of pestiviruses in their natural host, animal experiments were conducted with mutant V(pA/C-346-d) (C346-d in tables). Virus recovered from the CSFV full length clone without mutation (V(pA/CSFV)) served as a positive control (C-WT in tables). For each mutant three piglets (breed: German landrace; about 25kg body weight) were used. The infection dose was 1x10⁵ TCID₅₀ per animal; two thirds of the inoculate was administered intranasally (one third in each nostril), one third intramuscularly. The two groups were housed in separate isolation units. Blood was taken from the animals two times before infection and on days 3, 5, 7, 10, 12 and 14. In addition, temperature was recorded daily (Figure 2). The animals infected with the wild type virus showed typical symptoms of classical swine fever like fever, ataxia, anorexia, diarrhea, central nervous disorders, hemorrhages in the skin (Table 3a). Virus could be recovered form the blood on days 3 (animal #68) and on days 5, 7, 10, 14 (animals #68, #78, #121) (Table 3b) The animals were killed in a moribund stage at day 14 post infection. At this time, no virus neutralizing antibodies could be detected.

In contrast, the animals infected with the mutant did not develop clinical symptoms (Table 3a). The temperature stayed normal (Figure 2) over the whole experimental period and the animals never stopped taking up food. At no time virus could be recovered from the blood. Nevertheless, the animals were clearly infected and the virus most likely replicated since all animals developed neutralizing antibodies (Table 3c).

**Table 3a: Clinical signs after test infection:**

| Animal experiment 1 | | | | | | | | | |
|---|---|---|---|---|---|---|---|---|---|
| Anim. No.: | infected with | clinical signs | | | | | | | |
| | | fever | diarrhea | CNS disorders | anorexia | hemorrhages in skin | apathia | moribund at day of euthanasia | Hemorrhages in organs at necropsy |
| #68 | C-WT | + | + | + | + | + | + | + | + |
| #78 | C-WT | + | + | + | + | + | + | + | + |
| #121 | C-WT | + | + | + | + | + | + | + | + |
| #70 | C-346-d | - | - | - | - | - | - | - | n.a. |
| #72 | C-346-d | - | - | - | - | - | - | - | n.a. |
| #74 | C-346-d | - | - | - | - | - | - | - | n.a. |

### Description of Table 3a:

6 piglets (German land race; about 25kg body weight) in two groups (each group was housed separately) were included in the study. 3 animal were infected with CSFV-WT (1·10⁵ TCID₅₀) and 3 animals with C-346-d (1·10⁵ TCID₅₀). Rectal temperature and clinical signs were recorded and summarized as detailed in the table; n.a.: no necropsy was performed.

**Table 3b: Blood cell viremia after test infection**

| Animal experiment 1 | | | | | | |
|---|---|---|---|---|---|---|
| Animal number | infected with | viremia at days post infection | | | | |
| | | 3 | 5 | 7 | 10 | 14 |
| #68 | C-WT | + | + | + | + | + |
| #78 | C-WT | - | + | + | + | + |
| #121 | C-WT | - | + | + | + | + |
| #70 | C-346-d | - | - | - | - | - |
| #72 | C-346-d | - | - | - | - | - |
| #74 | C-346-d | - | - | - | - | - |

### Description of Table 3b:

Blood cell viremia was detected by cocultivation of blood with PK15 cells. After incubation at 37°C for 72h cells were washed with PBS, fixed with ice cold acetone/methanol and analyzed for infection by immunofluorescence with a monoclonal antibody specific for glycoprotein E2 (mAb A18, Weiland et al. 1990).

**Table 3c: Development of CSFV specific serum neutralization titer**

| days p.i. | -3 | 0 | 17 | 25 | 69 | 76 | 79 | 87 |
|---|---|---|---|---|---|---|---|---|
| pig # 70 | - | - | 1:18 | 1:162 | 1:162 | 1:162 | 1:486 | 1:1458 |
| pig # 72 | - | - | 1:18 | 1:54 | 1:486 | 1:1458 | 1:1458 | 1:4374 |
| pig # 74 | - | - | 1:6 | 1:54 | 1:162 | 1:162 | 1:486 | 1:1458 |

### Description of Table 3c:

Antibody titers of pigs infected with virus mutant C-346-d determined at different time points during the animal experiment:
50 µl of the diluted serum were mixed with 50 µl of medium containing 30 TCID₅₀ of virus (CSFV Alfort/Tübingen). After 90 minutes incubation at 37°C, 100 µl of cells (1.5x10⁴ cells) were added and the mixture was seeded in 96 well plates. After 72 h the cells were fixed with ice cold acetone/methanol and analyzed for infection by immunofluorescence with a monoclonal antibody specific for glycoprotein E2 (mAb A18, Weiland et al. 1990). On day 69 post infection the animals were challenged with 2x10⁵ TCID₅₀ of CSFV strain Eystrup. The table gives the highest serum dilution resulting in complete neutralization of input virus.

### Example 4 Induction of protective immunity by infection with RNase negative virus

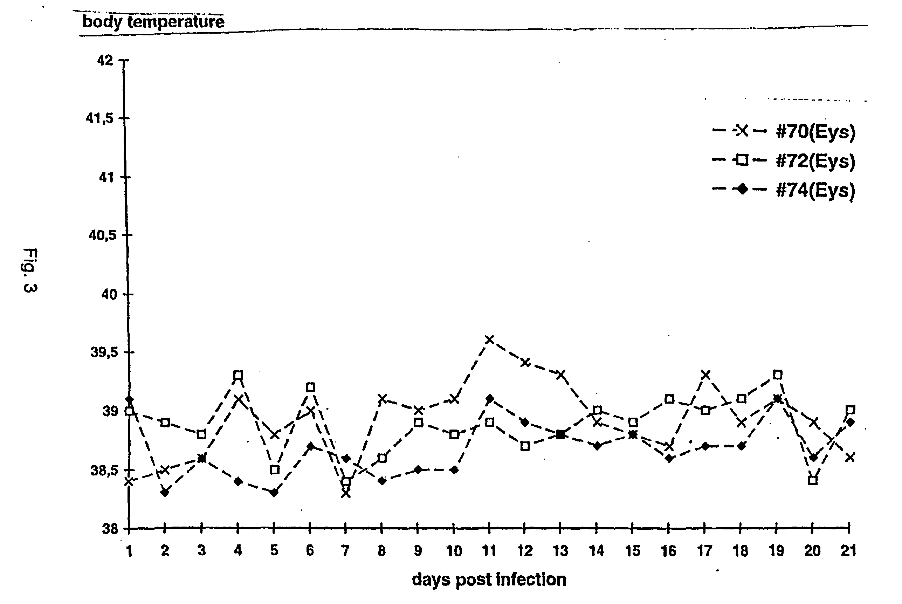

To analyze whether the infection with the mutant virus had led to a protective immunity, a challenge experiment was conducted about 9 weeks after the infection with the CSFV mutant using a highly pathogenic heterologous CSFV strain (strain Eystrup, originated from Behring). 2x10⁵ TCID₅₀ of virus was used for the infection. This amount of virus was found to be sufficient to induce lethal disease in several preceeding experiments (König, 1994). However, the animals previously infected with the CSFV RNase mutant did not show symptoms of disease after challenge infection. Neither fever (Figure 3) nor viremia could be detected but an increase in neutralizing antibodies indicated productive infection and repiication of the challenge virus.

### Example 5 Confirmation of attenuation principle

To show, that the observed attenuation of the mutant virus is indeed due to the deletion of the histidine at position 346 of the polyprotein and not a consequence of an unidentified second site mutation, the wild type sequence was restored by exchange of a 1.6 kb Xhol/Ndel fragment of the full length clone pA/C-346-d against the corresponding fragment of pA/CSFV displaying the wild type sequence. The fragment excised from pA/C-346-d was analyzed by nucleotide sequencing for mutations. Except for the deletion of the triplet coding for histidine 346 of the polyprotein, no difference with regard to the wild type sequence was found. From the cDNA construct with the rescued mutant, virus V(pA/C-346-d/Rs) could be recovered that grew equally well as wild type virus and showed equivalent RNase activity (Table 2A).

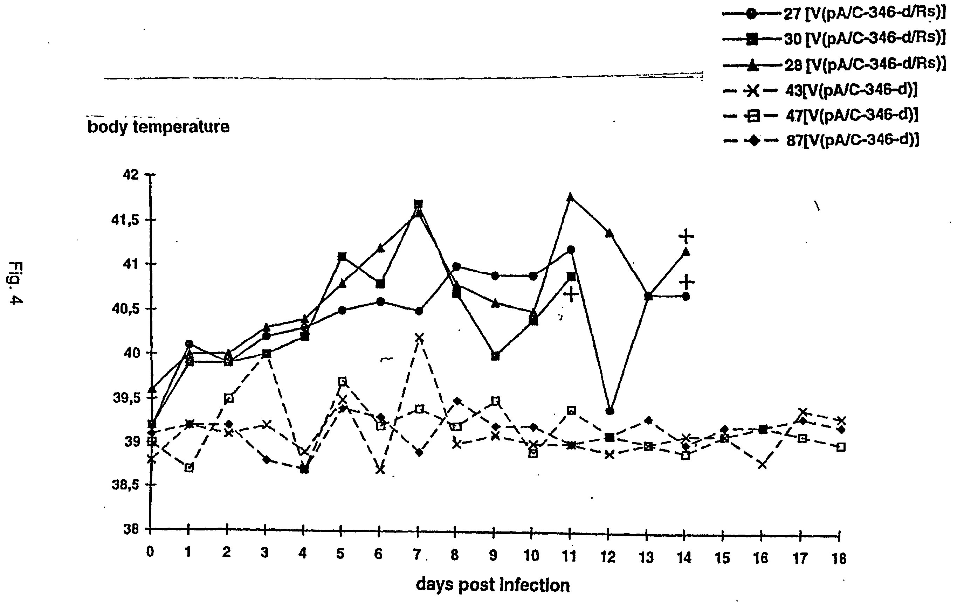

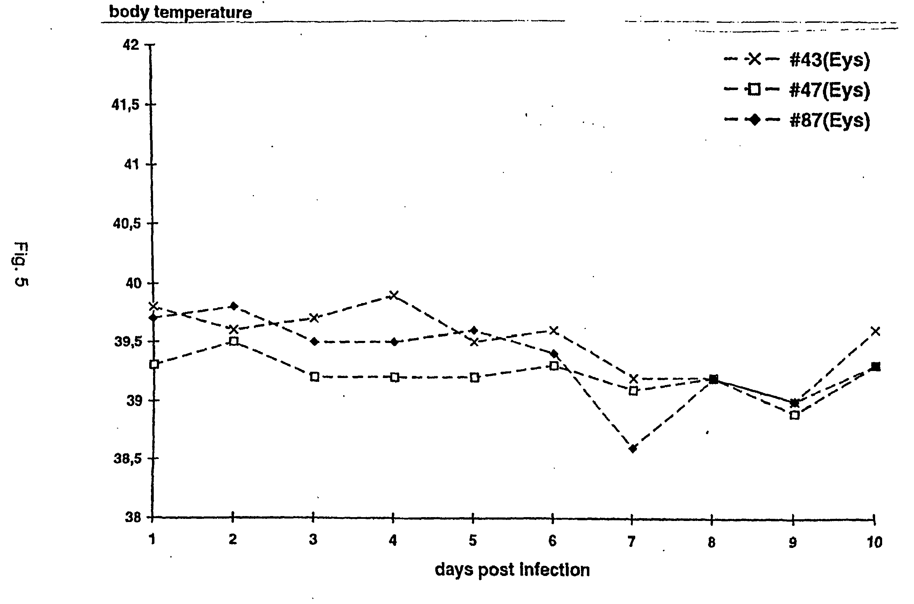

In a second animal experiment, the rescued virus was used for infection of pigs. As a control, the deletion mutant was used. Again, two groups consisting of three animals were used. As the animals were younger (German landrace, about 20kg) than those in the first experiment, 5x10⁴ TCID₅₀ of virus were used for infection this time. Again, the animals infected with the mutant showed no clinical signs (Table 5, Figure 4). Only one animal had fever for one day. Nevertheless, these animals developed neutralizing antibodies and were protected against a lethal CSFV challenge. Challenge was again performed by infection with 2x10⁵ TCID₅₀ of challenge strain Eystrup. The animals did not show clinical signs after challenge and the temperature stayed normal (Figure 5). In contrast to the pigs infected with the deletion mutant, the animals inoculated with the rescued wild type virus developed fatal classical swine fever. One animal had to be killed 11 days after infection, the other two 3 days later. All animals showed typical symptoms of classical swine fever, i.e. fever, diarrhea, annorexia, and pathological signs like hemorrhages in different organs including the kidney.

**Table 5a: Clinical signs after test infection**

| Animal experiment 2 | | | | | | | | | |
|---|---|---|---|---|---|---|---|---|---|
| Anim. No.: | Infected with | clinical signs | | | | | | | |
| | | fever | diarrhea | CNS disorders | anorexia | hemorrhages in skin | apathia | moribund at day of euthanasia | hemorrhages in organs at necropsy |
| #43 | C-346-d | +* | - | - | - | - | - | - | n.a. |
| #47 | C-346-d | - | - | - | - | - | - | - | n.a. |
| #87 | C-346-d | - | - | - | - | - | - | - | n.a. |
| #27 | C-346-d/RS | + | + | + | + | - | + | + | + |
| #28 | C-346-d/RS | + | + | + | + | - | + | + | + |
| #30 | C-346-d/RS | + | + | + | + | - | + | + | + |
| | | * fever for only 1 day | | | | | | | |

### Table 5a:

6 piglets (German land race; about 20 kg body weight) in two groups (each group was housed separately under isolation conditions) were included in the study. 3 animal were infected with mutant C-346-d (5·10⁴ TCID₅₀) and 3 animals with C-346-d/RS (5·10⁴ TCID₅₀). C-346-d/RS was derived from mutant C-346-d by restoring the wild type sequence of E^{RNS} gene. Rectal temperature and clinical signs were recorded and summarized; n.a.: no necropsy was performed.

**Table 5b Diagnostic RNAse test with viruses recovered from infected animals during viremia**

| | Alfort | animal #3 C-297-K | animal #5 C-297-K | animal #27 C -346-d/RS | animal #28 C -346-d/RS | animal #30 C -346-d/RS | Control |
|---|---|---|---|---|---|---|---|
| OD₂₆₀ | 1.84 | 0.60 | 0.56 | 1.84 | 1.93 | 1.94 | 0.49 |

Viruses recovered form the blood of animals 3 and 5 at day 5 post infection and of animals 27, 28 and 30 of animal experiment #2 (described in example 5) at day 7 post infection were propagated in tissue culture, titrated and tested for RNase activity as described above. Non-infected PK15 cells and cells (control) infected with wild type CSFV (Alfort) served as controls. Animals 3 and 5 had been infected with mutant C-297-K, whereas animals 27, 28 and 30 had been infected with mutant C-346-d/RS, as indicated in the table.

### Example 6 Effects of double mutation within E^{RNS}

To test the effects of a double mutation within E^{RNS} on the ability of the respective virus to replicate in its natural host and on pathogenicity, an animal experiment was conducted with mutant V(pA/C-297-L/346-L). Virus recovered from the CSFV full length clone without mutation (V(pA/CSFV) served as a positive control. For each mutant three piglets (breed: German land race; about 25 kg body weight) were used.

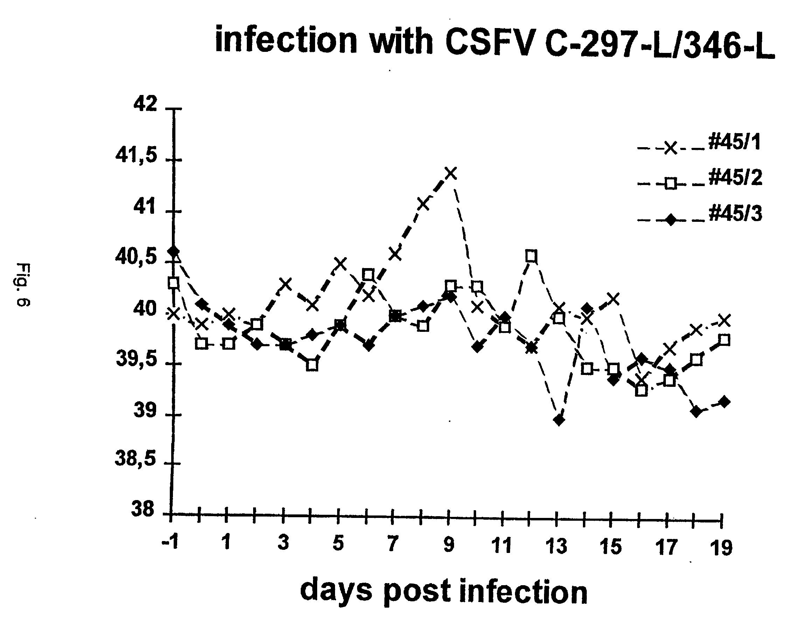

The infection dose was 1x10⁵ TCID₅₀ per animal; two thirds of the inoculate was administered intra-nasally (one third in each nostril), one third intramuscularly. Blood was taken from the animals before infection (day 0) and on days 5, 8, 12 and 20. In addition, temperature was recorded daily (Fig. 6). The animals infected with the double mutant did not develop any clinical symptoms, and the animals never stopped taking up food. The animals showed no fever over the whole experimental period (animals 45/2 and 45/3) except animal 45/1 on day 8, probably due to bacterial infection caused by injury of the right hind leg. After treatment of this animal with an antibiotic on day 10, temperature returned to normal values within one day (Fig. 6). For all animals virus was recovered from the blood on day 5 whereas no viremia was detected at later time points (Table 6a). All animals developed neutralizing antibodies (Table 6b). For animal 45/1 the neutralization titer was again determined about 4.5 months p.i. and was found to be 1:4374. Thus, the infection with the double mutant resulted in long lasting immunological memory.

**Table 6a Test for viremia**

| Days p.i. | 5 | 8 | 12 |
|---|---|---|---|
| Pig 45/I | + | - | - |
| Pig 45/II | + | - | - |
| Pig 45/III | + | - | - |

**Table 6b Neutralization titers**

| Animal | day 0 | day 20 p.i. |
|---|---|---|
| 45/1 | - | 1:128 |
| 45/2 | - | 1:256 |
| 45/3 | - | 1:256 |

### Example 7 Immunogenicity and attenuation principle of the BVDV virus "B-346-d"

This experiment was designed to investigate the attenuation principle as well as the immunogenicity of the BVDV virus 'B-346-d' recovered from pA/B-346-d by comparing it with the 'B-WT' virus recovered from pA/BVDV/Ins-. The virus ,B-346-d' is of course mutated in original BVDV position 349 but named "B-346" to indicate the position relative to the CSFV Alfort position 346 of figure 1.

Three groups of BVDV seronegative animals of 3-6 months of age were selected. Groups 1 and 2 comprised 5 animals each while group 3 comprised 3 animals.

Animals of group 1 and 2 were infected by administration of 2x10⁶ TCID₅₀ of B-346-d (group 1) or B-WT (group 2) in a volume of 5 ml per route. Animals were infected intra-muscularly (gluteal muscle), intranasally and subcutaneously (over scapula).

Over a period of 14 days after infection, viremia in both groups was monitored through parameters like blood cell viremia and virus shedding in nasal swabs. In addition, clinical parameters like rectal temperatures, white blood cell counts and general health parameters were monitored.

The protective immunity against an infection with an antigenetically heterologous and virulent BVDV-isolate (#13) was investigated by challenge infection 77 days after infection of the animals of group 1 with B-346-d. Animals of group 3 served as challenge control and were infected according to the procedure for the animals of group 1 with the virulent BVDV-isolate. The BVDV virus (#13) belongs to a different antigenetic group (type II), whereas the B-346-d virus belongs to the antigenetic group (type I) according to the classification described by (Pellerin, C. et. al., 1994).

Animals of group 1 and 3 got infected by administration of 2x10⁶ TCID₅₀ of BVDV isolate (#13) in a volume of 5 ml per route. Animals were infected via the intra-muscular (gluteal muscle), intra-nasal and subcutaneous route (over Scapula). Over a period of 14 days after infection viremia in both groups was monitored by parameters like blood cell viremia and virus shedding in nasal swabs. In addition, clinical parameters like rectal temperatures, white blood cell counts and general health parameters were monitored.

After infection with B-346-d animals did not show any typical clinical symptoms of a BVDV infection such as rectal temperature increase (Table 7a), or any respiratory clinical symptomes (not shown).

The reduced blood cell viremia (Table 7b) and virus shedding in nasal swabs (Table 7c) did clearly indicate an attenuation of B-346-d compared to B-WT.

The virulent BVDV isolate #13 did induce in the animals of group 3 a strong viremia with typical signs of a BVDV infection, like rectal temperature increase over a period of several days (Table 7d), strong leucopenia (Table 7e), extended blood cell viremia (Table 7f) and virus shedding in nasal swab fluid (Table 7g). In contrast, animals of group 1, which had been vaccinated by infection with B-346-d, did show almost no clinical symptoms typical for a BVDV infection after the challenge infection with the virulent BVDV isolate #13. There was no significant increase in rectal temperatures after infection (Table 7d). The observed leucopenia was very marginal with regard to magnitude and duration (Table 7e). No BVDV could be isolated from the blood (Table 7f) and for only one animal virus shedding in nasal swab exudate could be detected (Table 7g).

Therefore, infection with B-346-d induces a strong immunity which clearly reduces clinical signs, virus shedding and blood cell viremia after challenge infection with a heterologous BVDV isolate.

**Table 7a: Mean rectal temperatures in group 1 (B-346-d) and 2 (B-WT)**

| Day of study: | 0 | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|
| Group 1 | 38,8 | 39,1 | 39,0 | 38,7 | 38,8 | 38,7 | 38,7 | 38,5 | 38,7 | 38,5 | 38,5 | 38,5 | 38,4 | 38,9 | 38,7 |
| Group 2 | 38,8 | 39,0 | 38,9 | 38,6 | 38,6 | 38,7 | 38,6 | 38,4 | 39,1 | 38,4 | 38,7 | 38,6 | 38,7 | 38,6 | 38,6 |

Animals of group 1 were infected at day 0 with 6x10⁶TCID₅₀ B-346-d, whereas animals of group 2 were infected with 6x10⁶TCID₅₀ B-WT.

**Fig. 7b: Blood cell viremia of groups 1 and 2**

| Group | Animal | First day nasal shedding recorded | Final day nasal shedding recorded | Recorded duration of nasal shedding (days) | Mean duration of group (days) |
|---|---|---|---|---|---|
| 1 | 1 | 6 | 6 | 1 | 1,4 |
| | 2 | 4 | 6 | 2 | |
| | 3 | 5 | 5 | 1 | |
| | 4 | - | - | 0 | |
| | 5 | 6 | 9 | 3 | |
| 2 | 6 | 4 | 8 | 5 | 4,4 |
| | 7 | 4 | 7 | 4 | |
| | 8 | 4 | 7 | 4 | |
| | 9 | 4 | 7 | 4 | |
| | 10 | 4 | 8 | 5 | |

EDTA blood was sampled daily up to day 10 post infection with B-346-d and B-WT, respectively. 0.2 ml of blood were added to each of 3 cultures of calf testis (Cte) cells with medium containing heparin (1 unit/ml to prevent clotting). After overnight incubation inoculum/medium was replaced with fresh medium without heparin. After incubation for 4 to 6 days, BVDV infected cells were detected by immunefluorescence with a polyclonal serum specific for BVDV.

Negative cultures were frozen and subsequently thawed. 0.2 ml thereof were passed to a second passage on Cte cells to confirm the absence of BVDV.

**Table 7c: Virus shedding in nasal fluid:**

| Group | Animal | First day nasal shedding recorded | Final day nasal shedding recorded | Number of days virus detected in exudate | Mean number of days detected virus per group |
|---|---|---|---|---|---|
| 1 | 1 | 4 | 8 | 4 | 2,6 |
| | 2 | 6 | 6 | 1 | |
| | 3 | 4 | 4 | 1 | |
| | 4 | 5 | 7 | 3 | |
| | 5 | 3 | 6 | 4 | |
| 2 | 6 | 6 | 8 | 3 | 3,6 |
| | 7 | 5 | 7 | 3 | |
| | 8 | 5 | 8 | 4 | |
| | 9 | 5 | 6 | 2 | |
| | 10 | 3 | 9 | 6 | |

Nasal exudate was centrifuged (1000g) to remove gross debris and contaminants. Supernatant fluid was removed and 0,2 ml were seeded to each of three cell cultures. After overnight incubation the inoculum/medium was replaced with 2 ml of fresh medium. After incubation for 4-6 days, BVDV infected cells were detected by immunofluorescence with a polyclonal serum specific for BVDV.

**Table 7d: Mean rectal temperatures of groups 1 and 3**

| Day of study: | -2 | -1 | 0 | 1 | 2 | 3 | 4 | 5 | 6 | 7 | 8 | 9 | 10 | 12 | 14 |
|---|---|---|---|---|---|---|---|---|---|---|---|---|---|---|---|
| Group 1 | 38,4 | 38,6 | 38,5 | 38,5 | 38,6 | 38,4 | 38,4 | 38,4 | 38,3 | 38,4 | 38,4 | 38,4 | 38,4 | 38,4 | 38,5 |
| Group 3 | 38,8 | 39,1 | 38,8 | 39,1 | 39,4 | 39,7 | 40,2 | 40,2 | 40,4 | 41,3 | 40,2 | 40,1 | 40,2 | 40,8 | 40,4 |

Rectal temperatures were recorded up to 16 days after challenge infection. Animals of group 1 and 3 were infected by 6x10⁶ TCID₅₀ of the virulent BVDV isolate #13.

**Table 7e: Mean white blood cell counts**

| Day of study: | -2 | -1 | 0 | 1 | 2 | 3 | 4 | 5 | 6 | 7 | 8 | 9 | 10 | 12 | 14 |
|---|---|---|---|---|---|---|---|---|---|---|---|---|---|---|---|
| Group 1 | 11,9 | 11,9 | 11,3 | 10,8 | 9,2 | 8,2 | 8,9 | 9,9 | 11,2 | 11,6 | 11,6 | 10,6 | 10,8 | 10,8 | 9,4 |
| Group 3 | 11,7 | 15,8 | 13,8 | 11,1 | 7,7 | 9,8 | 7,4 | 6,8 | 7,5 | 8,7 | 7,0 | 8,1 | 6,2 | 6,4 | 6,2 |

EDTA blood cell samples were taken daily from day -2 to 14 post challenge from each animal in both groups. Counts of white blood cells in EDTA blood samples were determined using a Sysmex Micro-Cell Counter F800.

**Table 7f: BVDV isolated from blood samples**

| Group | Animal | First day virus detected in blood | Final day virus detected in blood | Recorded duration of virus in blood (days) | Mean duration (days) |
|---|---|---|---|---|---|
| 1 | 1 | - | - | 0 | 0 |
| | 2 | - | - | 0 | |
| | 3 | - | - | 0 | |
| | 4 | - | - | 0 | |
| | 5 | - | - | 0 | |
| 3 | 11 | 3 | 10 | 8 | 9,7 |
| | 12 | 3 | 14 | 12 | |
| | 13 | 3 | 9 | 9 | |

EDTA blood was sampled daily up to day 10 post challenge. 0.2 ml of blood were added to each of 3 cultures of calf testis (Cte) cells with medium containing heparin (1 unit/ml to prevent clotting). After overnight incubation inoculum/medium was replaced with fresh medium without heparin. After incubation for 4 to 6 days cells BVDV infected cells were detected by immunefluoreszence with a polyclonal serum specific for BVDV.

Negative cultures were frozen and subsequently thawed. 0.2 ml thereof were passed to a second passage on Cte cells to confirm the absence of BVDV.

**Table 7g: Virus shedding in nasal fluid**

| Group | Animal | First day nasal shedding recorded | Final day nasal shedding recorded | Recorded duration of nasal shedding (days) | Mean duration (days, **per group**) |
|---|---|---|---|---|---|
| 1 | 1 | 3 | 4 | 2 | 0,8 |
| | 2 | - | - | 0 | |
| | 3 | - | - | 0 | |
| | 4 | - | - | 0 | |
| | 5 | 4 | 5 | 2 | |
| 3 | 11 | 3 | 14 | 12 | 10 |
| | 12 | 3 | 14 | 12 | |
| | 13 | 3 | 8 | 6 | |

Nasal exudate was centrifuged (1000g) to remove gross debris and contaminants. Supernatant fluid was removed and 0,2 ml thereof were seeded to each of three cell cultures. After overnight incubation the inoculum/medium was replaced with 2 ml of fresh medium. After incubation for 4-6 days BVDV infected cells were detected by immunefluorescence with a polyclonal serum specific for BVDV.

### Example 8 Discrimination between C-346-d and CSFV without deletion of the histidine codon 346 by RT-PCR

The RNA sequence coding for the conserved RNase motif in CSFV glycoprotein E^{RNS} is highly conserved. Among all known CSFV sequences no nucleotide exchanges were found in the region corresponding to residues 1387 to 1416 of the published sequence of the CSFV Alfort strain (Meyers et al., 1987). Thus, oligonucleotide primers derived from this conserved region of the genome can be used in an RT-PCR assay for detection of all CSFV isolates (see figure 7). In consequence, the absence of the triplet coding for histidine 346 (nucleotides 1399-1401) could be detected by an RT-PCR assay with an appropriately designed primer. Different oligonucleotides covering the conserved region were synthesized that either contained the histidine codon or not. These oligonucleotides served as upstream primers in RT-PCR reactions with oligonucleotide E^{RNS}-Stop as downstream primer. RNA purified from tissue culture cells infected with C-346-d, C-WT, C-346-L or C-346-K, respectively, were used as templates. Reverse transcription of 2 µg heat denatured RNA (2 min 92°C, 5 min on ice in 11.5 µl of water in the presence of 30 pMol reverse primer) was done after addition of 8 µl RT mix (125 mM Tris/HCl pH 8.3, 182.5 mM KCI, 7.5 mM MgCl₂, 25 mM dithiothreitol, 1.25 mM of each dATP, dTTP, dCTP, dGTP), 15 U of RNAguard (Pharmacia, Freiburg, Germany) and 50 U of Superscript (Life Technologies/BRL, Eggenstein, Germany) for 45 min at 37°C. After finishing reverse transcription, the tubes were placed on ice and 30 µl of PCR mix (8.3 mM Tris/HCl, pH8.3; 33.3 mM KCl; 2.2 mM MgCl₂; 0.42 mM of each dATP, dTTP, dCTP, dGTP; 0.17% TritonX100; 0.03% bovine serum albumine; 5 U of Taq polymerase (Appligene, Heidelberg, Germany) and 16.7% DMSO) were added. When primer Ol H+3 was used, the reaction mix for amplification contained no DMSO. Amplification was carried out in 36 cycles (30 sec 94°C; 30 sec 57°C; 45 sec 74°C). 1 µl of amplification reaction was loaded on a 1% agarose gel, the amplified products were separated by electrophoresis, and stained with ethidium bromide. As demonstrated in Fig. 7, primer pair Ol H-3/Ol E^{rns}Stop allowed to specifically amplify a band derived from RNA containing the deletion of codon 346 whereas with the other two primer combinations products containing codon 346 were amplified and no band was observed when the RNA with the deletion of this codon was used as a template.

### Primers for RT-PCR:

upstream:

downstream:

### Figure legends

**Figure 1: The first 495 amino acids as expressed by the Alfort strain of CSFV** The sequence listing shows the first 495 amino acids as expressed by the Alfort strain of CSFV (Meyers et al., 1989). One monomer of the glycoprotein E^{RNS} of said strain corresponds to the amino acids 268 to 494 as described by Rümenapf et al. (1993). Residues 295 to 307 and 338 to 357 representing the regions showing homology to plant and fungal RNases (Schneider et al., 1993) are underlined.
**Figure 2: Rectal temperature curve of animals after test infection**
   Daily rectal temperature was recorded from day 2 before till day 18 post infection. Rectal temperature curve is detailed for each animal of the group infected with the virus V(pA/CSFV) (continuous line) derived from plasmid pA/CSFV or with the virus V(pA/C-346-d) derived from plasmid pA/C-346-d (dotted line).
**Figure 3: Rectal temperature curve of animals after challenge infection**
   Daily rectal temperature was recorded at days 1-21 post challenge virus infection. Animals challenged with a lethal dosis of the CSFV challenge strain Eystrup had been infected with mutant C-346-d [V(pA/C-346-d)] 69 days in before as detailed in the text. Rectal temperature curve is detailed for each animal of the group challenged with 2x10⁵ TCID₅₀ from the CSFV challenge strain Eystrup
**Figure 4: Rectal temperature curve of animals after test infection**
   Daily rectal temperature was recorded at days 0-18 post infection. Rectal temperature curve is detailed for each animal of the two groups infected either with C-346-d [V(pA/C-346-d)] (dotted line) or with the restored virus C-346-d/RS [V(pA/C-346-d/Rs)] (continuous line).
**Figure 5: Rectal temperature after challenge infection animal experiment #2**
   Daily rectal temperature was recorded at days 1-10 post challenge virus infection. Animals challenged with a lethal dose (2x10⁵ TCID₅₀) of the CSFV challenge strain Eystrup had been infected with mutant C-346-d 37 days in before.
**Figure 6: Rectal temperature of animals treated with a double mutant according to example 6**
   Daily rectal temperature was recorded prior and post challenge virus infection with mutant V(pA/C-297-L/346-L).
**Figure 7: Discrimination between C-346-d and CSFV without deletion of the histidine codon 346 by RT-PCR according to example 8**
   a) Primer pair Ol H-3/Ol E^{rns}Stop allows to specifically amplify a band derived from RNA containing the deletion of codon 346 (C-346-d) as described in detail in example 8. In contrast, RNA, not containing said deletion does not interact with said primer pair (C-WT, C-346-L, C-346-K).
   b) And c) The other two primer combinations (OI H+2 and OI H+3) amplify bands derived from RNA that do not contain the deletion of codon 346 (OI H+2 and OI H+3). No band can be observed when RNA from the 346-deletion mutant C-346-d is used as a template.

## Claims

1. A method for detectably labeling pestiviruses **characterized in that** the RNase activity residing in glycoprotein E^{RNS} is inactivated, wherein said RNase activity is inactivated by deletions and/or mutations of at least one amino acid of said glycoprotein, with the proviso that the amino acids at position 297 and/or 346, as described in figure 1 for the CSFV Alfort strain in an exemplary manner or corresponding thereto in other strains, of said glycoprotein are not lysine, and wherein inactivation of said RNase activity residing in glycoprotein E^{RNS} results in an attenuated pestivirus.

2. The method of claim 1, wherein said deletions and/or mutations are located at the amino acids at position 295 to 307 and/or position 338 to 357, as described in figure 1 for the CSFV Alfort strain in an exemplary manner or corresponding thereto in other strains, of said glycoprotein.

3. The method according to any one of claims 1 to 2, wherein said RNase activity is inactivated by deletion or mutation of the amino acid at position 346, as described in figure 1 for the CSFV Alfort strain in an exemplary manner or corresponding thereto in other strains, of said glycoprotein.

4. The method according to any one of claims 1 to 3, wherein said RNase activity is inactivated by the deletion of the histidine residue at position 346, as described in figure 1 for the CSFV Alfort strain in an exemplary manner or corresponding thereto in other strains, of said glycoprotein.

## Patentansprüche

1. Verfahren zum nachweisbaren Markieren von Pestiviren, **dadurch gekennzeichnet, dass** die RNase-Aktivität, die in Glycoprotein E^{RNS} enthalten ist, inaktiviert wird, wobei die RNase-Aktivität durch Deletionen und/oder Mutationen von mindestens einer Aminosäure des Glycoproteins inaktiviert wird, mit der Maßgabe, dass es sich bei den Aminosäuren in Position 297 und/oder 346, die in Fig. 1 für den CSFV-Alfort-Stamm in beispielhafter Weise dargestellt sind oder diesen in anderen Stämmen entsprechen, des Glycoproteins nicht um Lysin handelt, und wobei die Inaktivierung der RNase-Aktivität, die in Glycoprotein E^{RNS} enthalten ist, zu einem abgeschwächten Pestivirus führt.

2. Verfahren nach Anspruch 1, wobei die Deletionen und/oder Mutationen sich an den Aminosäuren in Position 295 bis 307 und/oder Position 338 bis 357, die in Fig. 1 für den CSFV-Alfort-Stamm in beispielhafter Weise dargestellt sind oder diesen in anderen Stämmen entsprechen, des Glycoproteins befinden.

3. Verfahren nach einem der Ansprüche 1 bis 2, wobei die RNase-Aktivität durch Deletion oder Mutation der Aminosäure in Position 346, die in Fig. 1 für den CSFV-Alfort-Stamm in beispielhafter Weise dargestellt ist oder dieser in anderen Stämmen entspricht, des Glycoproteins inaktiviert wird.

4. Verfahren nach einem der Ansprüche 1 bis 3, wobei die RNase-Aktivität durch Deletion des Histidin-Restes in Position 346, der in Fig. 1 für den CSFV-Alfort-Stamm in beispielhafter Weise dargestellt ist oder diesem in anderen Stämmen entspricht, des Glycoproteins inaktiviert wird.

## Revendications

1. Procédé pour marquer de manière détectable des pestivirus **caractérisé en ce que** l'activité RNase résidant dans la glycoprotéine E^{RNS} est inactivée, où ladite activité RNase est inactivée par des délétions et/ou des mutations d'au moins un aminoacide de ladite glycoprotéine, avec la condition que les aminoacides à la position 297 et/ou 346, comme décrit sur la figure 1 pour la souche CSFV Alfort à titre d'exemple ou d'une manière correspondant à celle-ci dans d'autres souches, de ladite glycoprotéine ne sont pas la lysine, et où l'inactivation de ladite activité RNase résidant dans la glycoprotéine E^{RNS} conduit à un pestivirus atténué.

2. Procédé selon la revendication 1 où lesdites délétions et/ou mutations sont localisées aux aminoacides à la position 295 à 307 et/ou à la position 338 à 357, comme décrit sur la figure 1 pour la souche CSFV Alfort à titre d'exemple ou d'une manière correspondant à celle-ci dans d'autres souches, de ladite glycoprotéine.

3. Procédé selon l'une quelconque des revendications 1 à 2 où ladite activité RNase est inactivée par délétion ou mutation de l'aminoacide à la position 346, comme décrit sur la figure 1 pour la souche CSFV Alfort à titre d'exemple ou d'une manière correspondant à celle-ci dans d'autres souches, de ladite glycoprotéine.

4. Procédé selon l'une quelconque des revendications 1 à 3 où ladite activité RNase est inactivée par la délétion du résidu histidine à la position 346, comme décrit sur la figure 1 pour la souche CSFV Alfort à titre d'exemple ou d'une manière correspondant à celle-ci dans d'autres souches, de ladite glycoprotéine.


### SEQUENCE LISTING

<110> Boehringer Ingelheim Vetmedica GmbH
<120> Attenuated pestiviruses
<130> 1-1039 B
<140> 02003408.8
   <141> 2002-02-14
<150> 02003408.8
   <151> 2002-02-14
<160> 28
<170> PatentIn Ver. 2.1
<210> 1
   <211> 20
   <212> DNA
   <213> Artificial Sequence
<220>
   <223> Description of Artificial Sequence: Primer
<400> 1
   aggagcttac ttgggatctg                                              20
<210> 2
   <211> 20
   <212> DNA
   <213> Artificial Sequence
<220>
   <223> Description of Artificial Sequence: Primer
<400> 2
   ggaacaaact tggatggtgt 20
<210> 3
   <211> 24
   <212> DNA
   <213> Artificial Sequence
<220>
   <223> Description of Artificial Sequence: Primer
<400> 3
   acaggagctt aaaagggatc tggc 24
<210> 4
   <211> 24
   <212> DNA
   <213> Artificial Sequence
<220>
   <223> Description of Artificial Sequence: Primer
<400> 4
   atggaacaaa aagggatggt gtaa 24
<210> 5
   <211> 24
   <212> DNA
   <213> Artificial Sequence
<220>
   <223> Description of Artificial Sequence: Primer
<400> 5
   gaatggaaca aaggatggtg taac 24
<210> 6
   <211> 30
   <212> DNA
   <213> Artificial Sequence
<220>
   <223> Description of Artificial Sequence: Primer
<400> 6
   catgaatgga acaaaggttg gtgcaactgg 30
<210> 7
   <211> 495
   <212> PRT
   <213> Artificial Sequence
<220>
   <223> Description of Artificial Sequence: CSFV mutant
<400> 7
   Met Glu Leu Asn His Phe Glu Leu Leu Tyr Lys Thr Ser Lys Gln Lys
   1               5                   10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145 150 155 160
   Cys Pro Leu Trp Val Thr Ser Cys Ser Asp Asp Gly Ala Ser Gly Ser
   165 170 175
   Lys Asp Lys Lys Pro Asp Arg Met Asn Lys Gly Lys Leu Lys Ile Ala
   180 185 190
   Pro Arg Glu His Glu Lys Asp Ser Lys Thr Lys Pro Pro Asp Ala Thr
   195 200 205
   Ile Val Val Glu Gly Val Lys Tyr Gln Ile Lys Lys Lys Gly Lys Val
   210 215 220
   Lys Gly Lys Asn Thr Gln Asp Gly Leu Tyr His Asn Lys Asn Lys Pro
   225 230 235 240
   Pro Glu Ser Arg Lys Lys Leu Glu Lys Ala Leu Leu Ala Trp Ala Val
   245 250 255
   Ile Thr Ile Leu Leu Tyr Gln Pro Val Ala Ala Glu Asn Ile Thr Gln
   260 265 270
   Trp Asn Leu Ser Asp Asn Gly Thr Asn Gly Ile Gln Arg Ala Met Tyr
   275 280 285
   Leu Arg Gly Val Asn Arg Gly Leu His Gly Ile Trp Pro Glu Lys Ile
   290 295 300
   Cys Lys Gly Val Pro Thr His Leu Ala Thr Asp Thr Glu Leu Lys Glu
   305 310 315 320
   Ile Arg Gly Met Met Asp Ala Ser Glu Arg Thr Asn Tyr Thr Cys Cys
   325 330 335
   Arg Leu Gln Arg His Glu Trp Asn Lys His Gly Trp Cys Asn Trp Tyr
   340 345 350
   Asn Ile Asp Pro Trp Ile Gln Leu Met Asn Arg Thr Gln Thr Asn Leu
   355 360 365
   Thr Glu Gly Pro Pro Asp Lys Glu Cys Ala Val Thr Cys Arg Tyr Asp
   370 375 380
   Lys Asn Thr Asp Val Asn Val Val Thr Gln Ala Arg Asn Arg Pro Thr
   385 390 395 400
   Thr Leu Thr Gly Cys Lys Lys Gly Lys Asn Phe Ser Phe Ala Gly Thr
   405 410 415
   Val Ile Glu Gly Pro Cys Asn Phe Asn Val Ser Val Glu Asp Ile Leu
   420 425 430
   Tyr Gly Asp His Glu Cys Gly Ser Leu Leu Gln Asp Thr Ala Leu Tyr
   435 440 445
   Leu Leu Asp Gly Met Thr Asn Thr Ile Glu Asn Ala Arg Gln Gly Ala
   450 455 460
   Ala Arg Val Thr Ser Trp Leu Gly Arg Gln Leu Ser Thr Ala Gly Lys
   465 470 475 480
   Lys Leu Glu Arg Arg Ser Lys Thr Trp Phe Gly Ala Tyr Ala Leu
   485 490 495
<210> 8
   <211> 492
   <212> PRT
   <213> Artificial Sequence
<220>
   <223> Description of Artificial Sequence: CSFV mutant
<400> 8
   Met Glu Leu Asn His Phe Glu Leu Leu Tyr Lys Thr Ser Lys Gln Lys
    1               5                  10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Ile Trp Pro Glu Lys Ile Cys Lys Gly
       290                 295                 300
   Val Pro Thr His Leu Ala Thr Asp Thr Glu Leu Lys Glu Ile Arg Gly
   305                 310                 315                 320
   Met Met Asp Ala Ser Glu Arg Thr Asn Tyr Thr Cys Cys Arg Leu Gln
                   325                 330                 335
   Arg His Glu Trp Asn Lys His Gly Trp Cys Asn Trp Tyr Asn Ile Asp
               340                 345                 350
   Pro Trp Ile Gln Leu Met Asn Arg Thr Gln Thr Asn Leu Thr Glu Gly
           355                 360                 365
   Pro Pro Asp Lys Glu Cys Ala Val Thr Cys Arg Tyr Asp Lys Asn Thr
       370                 375                 380
   Asp Val Asn Val Val Thr Gln Ala Arg Asn Arg Pro Thr Thr Leu Thr
   385                 390                 395                 400
   Gly Cys Lys Lys Gly Lys Asn Phe Ser Phe Ala Gly Thr Val Ile Glu
                   405                 410                 415
   Gly Pro Cys Asn Phe Asn Val Ser Val Glu Asp Ile Leu Tyr Gly Asp
               420                 425                 430
   His Glu Cys Gly Ser Leu Leu Gln Asp Thr Ala Leu Tyr Leu Leu Asp
           435                 440                 445
   Gly Met Thr Asn Thr Ile Glu Asn Ala Arg Gln Gly Ala Ala Arg Val
       450                 455                 460
   Thr Ser Trp Leu Gly Arg Gln Leu Ser Thr Ala Gly Lys Lys Leu Glu
   465                 470                 475                 480
   Arg Arg Ser Lys Thr Trp Phe Gly Ala Tyr Ala Leu
                   485                 490
<210> 9
   <211> 494
   <212> PRT
   <213> Artificial Sequence
<220>
   <223> Description of Artificial Sequence: CSFV mutant
<400> 9
   Met Glu Leu Asn His Phe Glu Leu Leu Tyr Lys Thr Ser Lys Gln Lys
    1               5                  10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
           50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu Gly Ile Trp Pro Glu Lys Ile Cys
       290                 295                 300
   Lys Gly Val Pro Thr His Leu Ala Thr Asp Thr Glu Leu Lys Glu Ile
   305                 310                 315                 320
   Arg Gly Met Met Asp Ala Ser Glu Arg Thr Asn Tyr Thr Cys Cys Arg
                   325                 330                 335
   Leu Gln Arg His Glu Trp Asn Lys His Gly Trp Cys Asn Trp Tyr Asn
               340                 345                 350
   Ile Asp Pro Trp Ile Gln Leu Met Asn Arg Thr Gln Thr Asn Leu Thr
           355                 360                 365
   Glu Gly Pro Pro Asp Lys Glu Cys Ala Val Thr Cys Arg Tyr Asp Lys
       370                 375                 380
   Asn Thr Asp Val Asn Val Val Thr Gln Ala Arg Asn Arg Pro Thr Thr
   385 390 395 400
   Leu Thr Gly Cys Lys Lys Gly Lys Asn Phe Ser Phe Ala Gly Thr Val
   405 410 415
   Ile Glu Gly Pro Cys Asn Phe Asn Val Ser Val Glu Asp Ile Leu Tyr
   420 425 430
   Gly Asp His Glu Cys Gly Ser Leu Leu Gln Asp Thr Ala Leu Tyr Leu
   435 440 445
   Leu Asp Gly Met Thr Asn Thr Ile Glu Asn Ala Arg Gln Gly Ala Ala
   450 455 460
   Arg Val Thr Ser Trp Leu Gly Arg Gln Leu Ser Thr Ala Gly Lys Lys
   465 470 475 480
   Leu Glu Arg Arg Ser Lys Thr Trp Phe Gly Ala Tyr Ala Leu
   485 490
<210> 10
   <211> 495
   <212> PRT
   <213> Artificial Sequence
<220>
   <223> Description of Artificial Sequence: CSFV mutant
<400> 10
   Met Glu Leu Asn His Phe Glu Leu Leu Tyr Lys Thr Ser Lys Gln Lys
    1               5                  10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu Lys Gly Ile Trp Pro Glu Lys Ile
       290                 295                 300
   Cys Lys Gly Val Pro Thr His Leu Ala Thr Asp Thr Glu Leu Lys Glu
   305                 310                 315                 320
   Ile Arg Gly Met Met Asp Ala Ser Glu Arg Thr Asn Tyr Thr Cys Cys
                   325                 330                 335
   Arg Leu Gln Arg His Glu Trp Asn Lys His Gly Trp Cys Asn Trp Tyr
               340                 345                 350
   Asn Ile Asp Pro Trp Ile Gln Leu Met Asn Arg Thr Gln Thr Asn Leu
           355                 360                 365
   Thr Glu Gly Pro Pro Asp Lys Glu Cys Ala Val Thr Cys Arg Tyr Asp
       370                 375                 380
   Lys Asn Thr Asp Val Asn Val Val Thr Gln Ala Arg Asn Arg Pro Thr
   385                 390                 395                 400
   Thr Leu Thr Gly Cys Lys Lys Gly Lys Asn Phe Ser Phe Ala Gly Thr
                   405                 410                 415
   Val Ile Glu Gly Pro Cys Asn Phe Asn Val Ser Val Glu Asp Ile Leu
               420                 425                 430
   Tyr Gly Asp His Glu Cys Gly Ser Leu Leu Gln Asp Thr Ala Leu Tyr
           435                 440                 445
   Leu Leu Asp Gly Met Thr Asn Thr Ile Glu Asn Ala Arg Gln Gly Ala
       450                 455                 460
   Ala Arg Val Thr Ser Trp Leu Gly Arg Gln Leu Ser Thr Ala Gly Lys
   465                 470                 475                 480
   Lys Leu Glu Arg Arg Ser Lys Thr Trp Phe Gly Ala Tyr Ala Leu
                   485                 490                 495
<210> 11
   <211> 495
   <212> PRT
   <213> Artificial Sequence
<220>
   <223> Description of Artificial Sequence: CSFV mutant
<400> 11
   Met Glu Leu Asn His Phe Glu Leu Leu Tyr Lys Thr Ser Lys Gln Lys
   1               5                   10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu Lys Gly Ile Trp Pro Glu Lys Ile
       290                 295                 300
   Cys Lys Gly Val Pro Thr His Leu Ala Thr Asp Thr Glu Leu Lys Glu
   305                 310                 315                 320
   Ile Arg Gly Met Met Asp Ala Ser Glu Arg Thr Asn Tyr Thr Cys Cys
                   325                 330                 335
   Arg Leu Gln Arg His Glu Trp Asn Lys Lys Gly Trp Cys Asn Trp Tyr
               340                 345                 350
   Asn Ile Asp Pro Trp Ile Gln Leu Met Asn Arg Thr Gln Thr Asn Leu
           355                 360                 365
   Thr Glu Gly Pro Pro Asp Lys Glu Cys Ala Val Thr Cys Arg Tyr Asp
       370                 375                 380
   Lys Asn Thr Asp Val Asn Val Val Thr Gln Ala Arg Asn Arg Pro Thr
   385                 390                 395                 400
   Thr Leu Thr Gly Cys Lys Lys Gly Lys Asn Phe Ser Phe Ala Gly Thr
                   405                 410                 415
   Val Ile Glu Gly Pro Cys Asn Phe Asn Val Ser Val Glu Asp Ile Leu
               420                 425                 430
   Tyr Gly Asp His Glu Cys Gly Ser Leu Leu Gln Asp Thr Ala Leu Tyr
           435                 440                 445
   Leu Leu Asp Gly Met Thr Asn Thr Ile Glu Asn Ala Arg Gln Gly Ala
       450                 455                 460
   Ala Arg Val Thr Ser Trp Leu Gly Arg Gln Leu Ser Thr Ala Gly Lys
   465                 470                 475                 480
   Lys Leu Glu Arg Arg Ser Lys Thr Trp Phe Gly Ala Tyr Ala Leu
                   485                 490                 495
<210> 12
   <211> 495
   <212> PRT
   <213> Artificial Sequence
<220>
   <223> Description of Artificial Sequence: CSFV mutant
<400> 12
   Met Glu Leu Asn His Phe Glu Leu Leu Tyr Lys Thr Ser Lys Gln Lys
    1               5                  10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
   85 90 95
   Val Tyr His Arg Ala Pro Leu Glu Phe Phe Asp Glu Ala Gln Phe Cys
   100 105 110
   Glu Val Thr Lys Arg Ile Gly Arg Val Thr Gly Ser Asp Gly Lys Leu
   115 120 125
   Tyr His Ile Tyr Val Cys Val Asp Gly Cys Ile Leu Leu Lys Leu Ala
   130 135 140
   Lys Arg Gly Thr Pro Arg Thr Leu Lys Trp Ile Arg Asn Phe Thr Asn
   145 150 155 160
   Cys Pro Leu Trp Val Thr Ser Cys Ser Asp Asp Gly Ala Ser Gly Ser
   165 170 175
   Lys Asp Lys Lys Pro Asp Arg Met Asn Lys Gly Lys Leu Lys Ile Ala
   180 185 190
   Pro Arg Glu His Glu Lys Asp Ser Lys Thr Lys Pro Pro Asp Ala Thr
   195 200 205
   Ile Val Val Glu Gly Val Lys Tyr Gln Ile Lys Lys Lys Gly Lys Val
   210 215 220
   Lys Gly Lys Asn Thr Gln Asp Gly Leu Tyr His Asn Lys Asn Lys Pro
   225 230 235 240
   Pro Glu Ser Arg Lys Lys Leu Glu Lys Ala Leu Leu Ala Trp Ala Val
   245 250 255
   Ile Thr Ile Leu Leu Tyr Gln Pro Val Ala Ala Glu Asn Ile Thr Gln
   260 265 270
   Trp Asn Leu Ser Asp Asn Gly Thr Asn Gly Ile Gln Arg Ala Met Tyr
   275 280 285
   Leu Arg Gly Val Asn Arg Ser Leu Lys Gly Ile Trp Pro Glu Lys Ile
   290 295 300
   Cys Lys Gly Val Pro Thr His Leu Ala Thr Asp Thr Glu Leu Lys Glu
   305 310 315 320
   Ile Arg Gly Met Met Asp Ala Ser Glu Arg Thr Asn Tyr Thr Cys Cys
   325 330 335
   Arg Leu Gln Arg His Glu Trp Asn Lys Leu Gly Trp Cys Asn Trp Tyr
   340 345 350
   Asn Ile Asp Pro Trp Ile Gln Leu Met Asn Arg Thr Gln Thr Asn Leu
   355 360 365
   Thr Glu Gly Pro Pro Asp Lys Glu Cys Ala Val Thr Cys Arg Tyr Asp
   370 375 380
   Lys Asn Thr Asp Val Asn Val Val Thr Gln Ala Arg Asn Arg Pro Thr
   385 390 395 400
   Thr Leu Thr Gly Cys Lys Lys Gly Lys Asn Phe Ser Phe Ala Gly Thr
   405 410 415
   Val Ile Glu Gly Pro Cys Asn Phe Asn Val Ser Val Glu Asp Ile Leu
   420 425 430
   Tyr Gly Asp His Glu Cys Gly Ser Leu Leu Gln Asp Thr Ala Leu Tyr
   435 440 445
   Leu Leu Asp Gly Met Thr Asn Thr Ile Glu Asn Ala Arg Gln Gly Ala
   450 455 460
   Ala Arg Val Thr Ser Trp Leu Gly Arg Gln Leu Ser Thr Ala Gly Lys
   465 470 475 480
   Lys Leu Glu Arg Arg Ser Lys Thr Trp Phe Gly Ala Tyr Ala Leu
   485 490 495
<210> 13
   <211> 495
   <212> PRT
   <213> Artificial Sequence
<220>
   <223> Description of Artificial Sequence: CSFV mutant
<400> 13
   Met Glu Leu Asn His Phe Glu Leu Leu Tyr Lys Thr Ser Lys Gln Lys
    1               5                  10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu Leu Gly Ile Trp Pro Glu Lys Ile
       290                 295                 300
   Cys Lys Gly Val Pro Thr His Leu Ala Thr Asp Thr Glu Leu Lys Glu
   305                 310                 315                 320
   Ile Arg Gly Met Met Asp Ala Ser Glu Arg Thr Asn Tyr Thr Cys Cys
                   325                 330                 335
   Arg Leu Gln Arg His Glu Trp Asn Lys His Gly Trp Cys Asn Trp Tyr
               340                 345                 350
   Asn Ile Asp Pro Trp Ile Gln Leu Met Asn Arg Thr Gln Thr Asn Leu
           355                 360                 365
   Thr Glu Gly Pro Pro Asp Lys Glu Cys Ala Val Thr Cys Arg Tyr Asp
       370                 375                 380
   Lys Asn Thr Asp Val Asn Val Val Thr Gln Ala Arg Asn Arg Pro Thr
   385                 390                 395                 400
   Thr Leu Thr Gly Cys Lys Lys Gly Lys Asn Phe Ser Phe Ala Gly Thr
                   405                 410                 415
   Val Ile Glu Gly Pro Cys Asn Phe Asn Val Ser Val Glu Asp Ile Leu
               420                 425                 430
   Tyr Gly Asp His Glu Cys Gly Ser Leu Leu Gln Asp Thr Ala Leu Tyr
           435                 440                 445
   Leu Leu Asp Gly Met Thr Asn Thr Ile Glu Asn Ala Arg Gln Gly Ala
       450                 455                 460
   Ala Arg Val Thr Ser Trp Leu Gly Arg Gln Leu Ser Thr Ala Gly Lys
   465                 470                 475                 480
   Lys Leu Glu Arg Arg Ser Lys Thr Trp Phe Gly Ala Tyr Ala Leu
                   485                 490                 495
<210> 14
   <211> 495
   <212> PRT
   <213> Artificial Sequence
<220>
   <223> Description of Artificial Sequence: CSFV mutant
<400> 14
   Met Glu Leu Asn His Phe Glu Leu Leu Tyr Lys Thr Ser Lys Gln Lys
   1               5                   10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu His Gly Ile Trp Pro Glu Lys Ile
       290                 295                 300
   Cys Lys Gly Val Pro Thr His Leu Ala Thr Asp Thr Glu Leu Lys Glu
   305                 310                 315                 320
   Ile Arg Gly Met Met Asp Ala Ser Glu Arg Thr Asn Tyr Thr Cys Cys
                   325                 330                 335
   Arg Leu Gln Gly His Glu Trp Asn Lys His Gly Trp Cys Asn Trp Tyr
   340 345 350
   Asn Ile Asp Pro Trp Ile Gln Leu Met Asn Arg Thr Gln Thr Asn Leu
   355 360 365
   Thr Glu Gly Pro Pro Asp Lys Glu Cys Ala Val Thr Cys Arg Tyr Asp
   370 375 380
   Lys Asn Thr Asp Val Asn Val Val Thr Gln Ala Arg Asn Arg Pro Thr
   385 390 395 400
   Thr Leu Thr Gly Cys Lys Lys Gly Lys Asn Phe Ser Phe Ala Gly Thr
   405 410 415
   Val Ile Glu Gly Pro Cys Asn Phe Asn Val Ser Val Glu Asp Ile Leu
   420 425 430
   Tyr Gly Asp His Glu Cys Gly Ser Leu Leu Gln Asp Thr Ala Leu Tyr
   435 440 445
   Leu Leu Asp Gly Met Thr Asn Thr Ile Glu Asn Ala Arg Gln Gly Ala
   450 455 460
   Ala Arg Val Thr Ser Trp Leu Gly Arg Gln Leu Ser Thr Ala Gly Lys
   465 470 475 480
   Lys Leu Glu Arg Arg Ser Lys Thr Trp Phe Gly Ala Tyr Ala Leu
   485 490 495
<210> 15
   <211> 495
   <212> PRT
   <213> Artificial Sequence
<220>
   <223> Description of Artificial Sequence: CSFV mutant
<400> 15
   Met Glu Leu Asn His Phe Glu Leu Leu Tyr Lys Thr Ser Lys Gln Lys
   1 5 10 15
   Pro Val Gly Val Glu Glu Pro Val Tyr Asp Thr Ala Gly Arg Pro Leu
   20 25 30
   Phe Gly Asn Pro Ser Glu Val His Pro Gln Ser Thr Leu Lys Leu Pro
   35 40 45
   His Asp Arg Gly Arg Gly Asp Ile Arg Thr Thr Leu Arg Asp Leu Pro
   50 55 60
   Arg Lys Gly Asp Cys Arg Ser Gly Asn His Leu Gly Pro Val Ser Gly
   65 70 75 80
   Ile Tyr Ile Lys Pro Gly Pro Val Tyr Tyr Gln Asp Tyr Thr Gly Pro
   85 90 95
   Val Tyr His Arg Ala Pro Leu Glu Phe Phe Asp Glu Ala Gln Phe Cys
   100 105 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu His Gly Ile Gly Pro Glu Lys Ile
       290                 295                 300
   Cys Lys Gly Val Pro Thr His Leu Ala Thr Asp Thr Glu Leu Lys Glu
   305                 310                 315                 320
   Ile Arg Gly Met Met Asp Ala Ser Glu Arg Thr Asn Tyr Thr Cys Cys
                   325                 330                 335
   Arg Leu Gln Arg His Glu Trp Asn Lys His Gly Trp Cys Asn Trp Tyr
               340                 345                 350
   Asn Ile Asp Pro Trp Ile Gln Leu Met Asn Arg Thr Gln Thr Asn Leu
           355                 360                 365
   Thr Glu Gly Pro Pro Asp Lys Glu Cys Ala Val Thr Cys Arg Tyr Asp
       370                 375                 380
   Lys Asn Thr Asp Val Asn Val Val Thr Gln Ala Arg Asn Arg Pro Thr
   385                 390                 395                 400
   Thr Leu Thr Gly Cys Lys Lys Gly Lys Asn Phe Ser Phe Ala Gly Thr
                   405                 410                 415
   Val Ile Glu Gly Pro Cys Asn Phe Asn Val Ser Val Glu Asp Ile Leu
               420                 425                 430
   Tyr Gly Asp His Glu Cys Gly Ser Leu Leu Gln Asp Thr Ala Leu Tyr
           435                 440                 445
   Leu Leu Asp Gly Met Thr Asn Thr Ile Glu Asn Ala Arg Gln Gly Ala
   450 455 460
   Ala Arg Val Thr Ser Trp Leu Gly Arg Gln Leu Ser Thr Ala Gly Lys
   465 470 475 480
   Lys Leu Glu Arg Arg Ser Lys Thr Trp Phe Gly Ala Tyr Ala Leu
   485 490 495
<210> 16
   <211> 494
   <212> PRT
   <213> Artificial Sequence
<220>
   <223> Description of Artificial Sequence: CSFV mutant
<400> 16
   Met Glu Leu Asn His Phe Glu Leu Leu Tyr Lys Thr Ser Lys Gln Lys
   1               5                   10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu His Gly Ile Trp Pro Glu Lys Ile
       290                 295                 300
   Cys Lys Gly Val Pro Thr His Leu Ala Thr Asp Thr Glu Leu Lys Glu
   305                 310                 315                 320
   Ile Arg Gly Met Met Asp Ala Ser Glu Arg Thr Asn Tyr Thr Cys Cys
                   325                 330                 335
   Arg Leu Gln Arg His Trp Asn Lys His Gly Trp Cys Asn Trp Tyr Asn
               340                 345                 350
   Ile Asp Pro Trp Ile Gln Leu Met Asn Arg Thr Gln Thr Asn Leu Thr
           355                 360                 365
   Glu Gly Pro Pro Asp Lys Glu Cys Ala Val Thr Cys Arg Tyr Asp Lys
       370                 375                 380
   Asn Thr Asp Val Asn Val Val Thr Gln Ala Arg Asn Arg Pro Thr Thr
   385                 390                 395                 400
   Leu Thr Gly Cys Lys Lys Gly Lys Asn Phe Ser Phe Ala Gly Thr Val
                   405                 410                 415
   Ile Glu Gly Pro Cys Asn Phe Asn Val Ser Val Glu Asp Ile Leu Tyr
               420                 425                 430
   Gly Asp His Glu Cys Gly Ser Leu Leu Gln Asp Thr Ala Leu Tyr Leu
           435                 440                 445
   Leu Asp Gly Met Thr Asn Thr Ile Glu Asn Ala Arg Gln Gly Ala Ala
       450                 455                 460
   Arg Val Thr Ser Trp Leu Gly Arg Gln Leu Ser Thr Ala Gly Lys Lys
   465                 470                 475                 480
   Leu Glu Arg Arg Ser Lys Thr Trp Phe Gly Ala Tyr Ala Leu
                   485                 490
<210> 17
   <211> 495
   <212> PRT
   <213> Artificial Sequence
<220>
   <223> Description of Artificial Sequence: CSFV mutant
<400> 17
   Met Glu Leu Asn His Phe Glu Leu Leu Tyr Lys Thr Ser Lys Gln Lys
   1 5 10 15
   Pro Val Gly Val Glu Glu Pro Val Tyr Asp Thr Ala Gly Arg Pro Leu
   20 25 30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu His Gly Ile Trp Pro Glu Lys Ile
       290                 295                 300
   Cys Lys Gly Val Pro Thr His Leu Ala Thr Asp Thr Glu Leu Lys Glu
   305                 310                 315                 320
   Ile Arg Gly Met Met Asp Ala Ser Glu Arg Thr Asn Tyr Thr Cys Cys
                   325                 330                 335
   Arg Leu Gln Arg His Glu Gly Asn Lys His Gly Trp Cys Asn Trp Tyr
               340                 345                 350
   Asn Ile Asp Pro Trp Ile Gln Leu Met Asn Arg Thr Gln Thr Asn Leu
           355                 360                 365
   Thr Glu Gly Pro Pro Asp Lys Glu Cys Ala Val Thr Cys Arg Tyr Asp
       370                 375                 380
   Lys Asn Thr Asp Val Asn Val Val Thr Gln Ala Arg Asn Arg Pro Thr
   385                 390                 395                 400
   Thr Leu Thr Gly Cys Lys Lys Gly Lys Asn Phe Ser Phe Ala Gly Thr
                   405                 410                 415
   Val Ile Glu Gly Pro Cys Asn Phe Asn Val Ser Val Glu Asp Ile Leu
               420                 425                 430
   Tyr Gly Asp His Glu Cys Gly Ser Leu Leu Gln Asp Thr Ala Leu Tyr
           435                 440                 445
   Leu Leu Asp Gly Met Thr Asn Thr Ile Glu Asn Ala Arg Gln Gly Ala
       450                 455                 460
   Ala Arg Val Thr Ser Trp Leu Gly Arg Gln Leu Ser Thr Ala Gly Lys
   465                 470                 475                 480
   Lys Leu Glu Arg Arg Ser Lys Thr Trp Phe Gly Ala Tyr Ala Leu
                   485                 490                 495
<210> 18
   <211> 492
   <212> PRT
   <213> Artificial Sequence
<220>
   <223> Description of Artificial Sequence: CSFV mutant
<400> 18
   Met Glu Leu Asn His Phe Glu Leu Leu Tyr Lys Thr Ser Lys Gln Lys
    1               5                  10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu His Gly Ile Trp Pro Glu Lys Ile
       290                 295                 300
   Cys Lys Gly Val Pro Thr His Leu Ala Thr Asp Thr Glu Leu Lys Glu
   305                 310                 315                 320
   Ile Arg Gly Met Met Asp Ala Ser Glu Arg Thr Asn Tyr Thr Cys Cys
                   325                 330                 335
   Arg Leu Gln Arg His Glu Trp Asn Trp Cys Asn Trp Tyr Asn Ile Asp
               340                 345                 350
   Pro Trp Ile Gln Leu Met Asn Arg Thr Gln Thr Asn Leu Thr Glu Gly
           355                 360                 365
   Pro Pro Asp Lys Glu Cys Ala Val Thr Cys Arg Tyr Asp Lys Asn Thr
       370                 375                 380
   Asp Val Asn Val Val Thr Gln Ala Arg Asn Arg Pro Thr Thr Leu Thr
   385                 390                 395                 400
   Gly Cys Lys Lys Gly Lys Asn Phe Ser Phe Ala Gly Thr Val Ile Glu
                   405                 410                 415
   Gly Pro Cys Asn Phe Asn Val Ser Val Glu Asp Ile Leu Tyr Gly Asp
               420                 425                 430
   His Glu Cys Gly Ser Leu Leu Gln Asp Thr Ala Leu Tyr Leu Leu Asp
           435                 440                 445
   Gly Met Thr Asn Thr Ile Glu Asn Ala Arg Gln Gly Ala Ala Arg Val
       450                 455                 460
   Thr Ser Trp Leu Gly Arg Gln Leu Ser Thr Ala Gly Lys Lys Leu Glu
   465                 470                 475                 480
   Arg Arg Ser Lys Thr Trp Phe Gly Ala Tyr Ala Leu
   485 490
<210> 19
   <211> 493
   <212> PRT
   <213> Artificial Sequence
<220>
   <223> Description of Artificial Sequence: CSFV mutant
<400> 19
   Met Glu Leu Asn His Phe Glu Leu Leu Tyr Lys Thr Ser Lys Gln Lys
    1               5                  10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                      80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu His Gly Ile Trp Pro Glu Lys Ile
       290                 295                 300
   Cys Lys Gly Val Pro Thr His Leu Ala Thr Asp Thr Glu Leu Lys Glu
   305                 310                 315                 320
   Ile Arg Gly Met Met Asp Ala Ser Glu Arg Thr Asn Tyr Thr Cys Cys
                   325                 330                 335
   Arg Leu Gln Arg His Glu Trp Asn Gly Trp Cys Asn Trp Tyr Asn Ile
               340                 345                 350
   Asp Pro Trp Ile Gln Leu Met Asn Arg Thr Gln Thr Asn Leu Thr Glu
           355                 360                 365
   Gly Pro Pro Asp Lys Glu Cys Ala Val Thr Cys Arg Tyr Asp Lys Asn
       370                 375                 380
   Thr Asp Val Asn Val Val Thr Gln Ala Arg Asn Arg Pro Thr Thr Leu
   385                 390                 395                 400
   Thr Gly Cys Lys Lys Gly Lys Asn Phe Ser Phe Ala Gly Thr Val Ile
                   405                 410                 415
   Glu Gly Pro Cys Asn Phe Asn Val Ser Val Glu Asp Ile Leu Tyr Gly
               420                 425                 430
   Asp His Glu Cys Gly Ser Leu Leu Gln Asp Thr Ala Leu Tyr Leu Leu
           435                 440                 445
   Asp Gly Met Thr Asn Thr Ile Glu Asn Ala Arg Gln Gly Ala Ala Arg
       450                 455                 460
   Val Thr Ser Trp Leu Gly Arg Gln Leu Ser Thr Ala Gly Lys Lys Leu
   465                 470                 475                 480
   Glu Arg Arg Ser Lys Thr Trp Phe Gly Ala Tyr Ala Leu
                   485                 490
<210> 20
   <211> 495
   <212> PRT
   <213> Artificial Sequence
<220>
   <223> Description of Artificial Sequence: CSFV mutant
<400> 20
   Met Glu Leu Asn His Phe Glu Leu Leu Tyr Lys Thr Ser Lys Gln Lys
    1               5                  10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
             35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu His Gly Ile Trp Pro Glu Lys Ile
       290                 295                 300
   Cys Lys Gly Val Pro Thr His Leu Ala Thr Asp Thr Glu Leu Lys Glu
   305                 310                 315                 320
   Ile Arg Gly Met Met Asp Ala Ser Glu Arg Thr Asn Tyr Thr Cys Cys
                   325                 330                 335
   Arg Leu Gln Arg His Glu Trp Asn Ala His Gly Trp Cys Asn Trp Tyr
               340                 345                 350
   Asn Ile Asp Pro Trp Ile Gln Leu Met Asn Arg Thr Gln Thr Asn Leu
           355                 360                 365
   Thr Glu Gly Pro Pro Asp Lys Glu Cys Ala Val Thr Cys Arg Tyr Asp
       370                 375                 380
   Lys Asn Thr Asp Val Asn Val Val Thr Gln Ala Arg Asn Arg Pro Thr
   385                 390                 395                 400
   Thr Leu Thr Gly Cys Lys Lys Gly Lys Asn Phe Ser Phe Ala Gly Thr
                   405                 410                 415
   Val Ile Glu Gly Pro Cys Asn Phe Asn Val Ser Val Glu Asp Ile Leu
               420                 425                 430
   Tyr Gly Asp His Glu Cys Gly Ser Leu Leu Gln Asp Thr Ala Leu Tyr
           435                 440                 445
   Leu Leu Asp Gly Met Thr Asn Thr Ile Glu Asn Ala Arg Gln Gly Ala
       450                 455                 460
   Ala Arg Val Thr Ser Trp Leu Gly Arg Gln Leu Ser Thr Ala Gly Lys
   465                 470                 475                 480
   Lys Leu Glu Arg Arg Ser Lys Thr Trp Phe Gly Ala Tyr Ala Leu
                   485                 490                 495
<210> 21
   <211> 493
   <212> PRT
   <213> Artificial Sequence
<220>
   <223> Description of Artificial Sequence: CSFV mutant
<400> 21
   Met Glu Leu Asn His Phe Glu Leu Leu Tyr Lys Thr Ser Lys Gln Lys
   1               5                   10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
   180 185 190
   Pro Arg Glu His Glu Lys Asp Ser Lys Thr Lys Pro Pro Asp Ala Thr
   195 200 205
   Ile Val Val Glu Gly Val Lys Tyr Gln Ile Lys Lys Lys Gly Lys Val
   210 215 220
   Lys Gly Lys Asn Thr Gln Asp Gly Leu Tyr His Asn Lys Asn Lys Pro
   225 230 235 240
   Pro Glu Ser Arg Lys Lys Leu Glu Lys Ala Leu Leu Ala Trp Ala Val
   245 250 255
   Ile Thr Ile Leu Leu Tyr Gln Pro Val Ala Ala Glu Asn Ile Thr Gln
   260 265 270
   Trp Asn Leu Ser Asp Asn Gly Thr Asn Gly Ile Gln Arg Ala Met Tyr
   275 280 285
   Leu Arg Gly Val Asn Arg Ser Leu His Gly Ile Trp Pro Glu Lys Ile
   290 295 300
   Cys Lys Gly Val Pro Thr His Leu Ala Thr Asp Thr Glu Leu Lys Glu
   305 310 315 320
   Ile Arg Gly Met Met Asp Ala Ser Glu Arg Thr Asn Tyr Thr Cys Cys
   325 330 335
   Arg Leu Gln Arg His Glu Trp Asn Lys Trp Cys Asn Trp Tyr Asn Ile
   340 345 350
   Asp Pro Trp Ile Gln Leu Met Asn Arg Thr Gln Thr Asn Leu Thr Glu
   355 360 365
   Gly Pro Pro Asp Lys Glu Cys Ala Val Thr Cys Arg Tyr Asp Lys Asn
   370 375 380
   Thr Asp Val Asn Val Val Thr Gln Ala Arg Asn Arg Pro Thr Thr Leu
   385 390 395 400
   Thr Gly Cys Lys Lys Gly Lys Asn Phe Ser Phe Ala Gly Thr Val Ile
   405 410 415
   Glu Gly Pro Cys Asn Phe Asn Val Ser Val Glu Asp Ile Leu Tyr Gly
   420 425 430
   Asp His Glu Cys Gly Ser Leu Leu Gln Asp Thr Ala Leu Tyr Leu Leu
   435 440 445
   Asp Gly Met Thr Asn Thr Ile Glu Asn Ala Arg Gln Gly Ala Ala Arg
   450 455 460
   Val Thr Ser Trp Leu Gly Arg Gln Leu Ser Thr Ala Gly Lys Lys Leu
   465 470 475 480
   Glu Arg Arg Ser Lys Thr Trp Phe Gly Ala Tyr Ala Leu
   485 490
<210> 22
   <211> 494
   <212> PRT
   <213> Artificial Sequence
<220>
   <223> Description of Artificial Sequence: CSFV mutant
<400> 22
   Met Glu Leu Asn His Phe Glu Leu Leu Tyr Lys Thr Ser Lys Gln Lys
    1               5                  10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu His Gly Ile Trp Pro Glu Lys Ile
       290                 295                 300
   Cys Lys Gly Val Pro Thr His Leu Ala Thr Asp Thr Glu Leu Lys Glu
   305 310 315 320
   Ile Arg Gly Met Met Asp Ala Ser Glu Arg Thr Asn Tyr Thr Cys Cys
   325 330 335
   Arg Leu Gln Arg His Glu Trp Asn Lys Gly Trp Cys Asn Trp Tyr Asn
   340 345 350
   Ile Asp Pro Trp Ile Gln Leu Met Asn Arg Thr Gln Thr Asn Leu Thr
   355 360 365
   Glu Gly Pro Pro Asp Lys Glu Cys Ala Val Thr Cys Arg Tyr Asp Lys
   370 375 380
   Asn Thr Asp Val Asn Val Val Thr Gln Ala Arg Asn Arg Pro Thr Thr
   385 390 395 400
   Leu Thr Gly Cys Lys Lys Gly Lys Asn Phe Ser Phe Ala Gly Thr Val
   405 410 415
   Ile Glu Gly Pro Cys Asn Phe Asn Val Ser Val Glu Asp Ile Leu Tyr
   420 425 430
   Gly Asp His Glu Cys Gly Ser Leu Leu Gln Asp Thr Ala Leu Tyr Leu
   435 440 445
   Leu Asp Gly Met Thr Asn Thr Ile Glu Asn Ala Arg Gln Gly Ala Ala
   450 455 460
   Arg Val Thr Ser Trp Leu Gly Arg Gln Leu Ser Thr Ala Gly Lys Lys
   465 470 475 480
   Leu Glu Arg Arg Ser Lys Thr Trp Phe Gly Ala Tyr Ala Leu
   485 490
<210> 23
   <211> 495
   <212> PRT
   <213> Artificial Sequence
<220>
   <223> Description of Artificial Sequence: CSFV mutant
<400> 23
   Met Glu Leu Asn His Phe Glu Leu Leu Tyr Lys Thr Ser Lys Gln Lys
    1               5                   10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
   100 105 110
   Glu Val Thr Lys Arg Ile Gly Arg Val Thr Gly Ser Asp Gly Lys Leu
   115 120 125
   Tyr His Ile Tyr Val Cys Val Asp Gly Cys Ile Leu Leu Lys Leu Ala
   130 135 140
   Lys Arg Gly Thr Pro Arg Thr Leu Lys Trp Ile Arg Asn Phe Thr Asn
   145 150 155 160
   Cys Pro Leu Trp Val Thr Ser Cys Ser Asp Asp Gly Ala Ser Gly Ser
   165 170 175
   Lys Asp Lys Lys Pro Asp Arg Met Asn Lys Gly Lys Leu Lys Ile Ala
   180 185 190
   Pro Arg Glu His Glu Lys Asp Ser Lys Thr Lys Pro Pro Asp Ala Thr
   195 200 205
   Ile Val Val Glu Gly Val Lys Tyr Gln Ile Lys Lys Lys Gly Lys Val
   210 215 220
   Lys Gly Lys Asn Thr Gln Asp Gly Leu Tyr His Asn Lys Asn Lys Pro
   225 230 235 240
   Pro Glu Ser Arg Lys Lys Leu Glu Lys Ala Leu Leu Ala Trp Ala Val
   245 250 255
   Ile Thr Ile Leu Leu Tyr Gln Pro Val Ala Ala Glu Asn Ile Thr Gln
   260 265 270
   Trp Asn Leu Ser Asp Asn Gly Thr Asn Gly Ile Gln Arg Ala Met Tyr
   275 280 285
   Leu Arg Gly Val Asn Arg Ser Leu His Gly Ile Trp Pro Glu Lys Ile
   290 295 300
   Cys Lys Gly Val Pro Thr His Leu Ala Thr Asp Thr Glu Leu Lys Glu
   305 310 315 320
   Ile Arg Gly Met Met Asp Ala Ser Glu Arg Thr Asn Tyr Thr Cys Cys
   325 330 335
   Arg Leu Gln Arg His Glu Trp Asn Lys Lys Gly Trp Cys Asn Trp Tyr
   340 345 350
   Asn Ile Asp Pro Trp Ile Gln Leu Met Asn Arg Thr Gln Thr Asn Leu
   355 360 365
   Thr Glu Gly Pro Pro Asp Lys Glu Cys Ala Val Thr Cys Arg Tyr Asp
   370 375 380
   Lys Asn Thr Asp Val Asn Val Val Thr Gln Ala Arg Asn Arg Pro Thr
   385 390 395 400
   Thr Leu Thr Gly Cys Lys Lys Gly Lys Asn Phe Ser Phe Ala Gly Thr
   405 410 415
   Val Ile Glu Gly Pro Cys Asn Phe Asn Val Ser Val Glu Asp Ile Leu
   420 425 430
   Tyr Gly Asp His Glu Cys Gly Ser Leu Leu Gln Asp Thr Ala Leu Tyr
   435 440 445
   Leu Leu Asp Gly Met Thr Asn Thr Ile Glu Asn Ala Arg Gln Gly Ala
   450 455 460
   Ala Arg Val Thr Ser Trp Leu Gly Arg Gln Leu Ser Thr Ala Gly Lys
   465 470 475 480
   Lys Leu Glu Arg Arg Ser Lys Thr Trp Phe Gly Ala Tyr Ala Leu
   485 490 495
<210> 24
   <211> 495
   <212> PRT
   <213> Artificial Sequence
<220>
   <223> Description of Artificial Sequence: CSFV mutant
<400> 24
   Met Glu Leu Asn His Phe Glu Leu Leu Tyr Lys Thr Ser Lys Gln Lys
   1               5                   10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
           210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu His Gly Ile Trp Pro Glu Lys Ile
       290                 295                 300
   Cys Lys Gly Val Pro Thr His Leu Ala Thr Asp Thr Glu Leu Lys Glu
   305                 310                 315                 320
   Ile Arg Gly Met Met Asp Ala Ser Glu Arg Thr Asn Tyr Thr Cys Cys
                   325                 330                 335
   Arg Leu Gln Arg His Glu Trp Asn Lys Leu Gly Trp Cys Asn Trp Tyr
               340                 345                 350
   Asn Ile Asp Pro Trp Ile Gln Leu Met Asn Arg Thr Gln Thr Asn Leu
           355                 360                 365
   Thr Glu Gly Pro Pro Asp Lys Glu Cys Ala Val Thr Cys Arg Tyr Asp
       370                 375                 380
   Lys Asn Thr Asp Val Asn Val Val Thr Gln Ala Arg Asn Arg Pro Thr
   385                 390                 395                 400
   Thr Leu Thr Gly Cys Lys Lys Gly Lys Asn Phe Ser Phe Ala Gly Thr
                   405                 410                 415
   Val Ile Glu Gly Pro Cys Asn Phe Asn Val Ser Val Glu Asp Ile Leu
               420                 425                 430
   Tyr Gly Asp His Glu Cys Gly Ser Leu Leu Gln Asp Thr Ala Leu Tyr
           435                 440                 445
   Leu Leu Asp Gly Met Thr Asn Thr Ile Glu Asn Ala Arg Gln Gly Ala
       450                 455                 460
   Ala Arg Val Thr Ser Trp Leu Gly Arg Gln Leu Ser Thr Ala Gly Lys
   465                 470                 475                 480
   Lys Leu Glu Arg Arg Ser Lys Thr Trp Phe Gly Ala Tyr Ala Leu
                   485                 490                 495
<210> 25
   <211> 495
   <212> PRT
   <213> Artificial Sequence
<220>
   <223> Description of Artificial Sequence: CSFV mutant
<400> 25
   Met Glu Leu Asn His Phe Glu Leu Leu Tyr Lys Thr Ser Lys Gln Lys
    1               5                  10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                 240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu His Gly Ile Trp Pro Glu Lys Ile
       290                 295                 300
   Cys Lys Gly Val Pro Thr His Leu Ala Thr Asp Thr Glu Leu Lys Glu
   305                 310                 315                 320
   Ile Arg Gly Met Met Asp Ala Ser Glu Arg Thr Asn Tyr Thr Cys Cys
                   325                 330                 335
   Arg Leu Gln Arg His Glu Trp Asn Lys His Gly Trp Cys Asn Trp Tyr
   340 345 350
   Asn Ile Asp Pro Trp Ile Gln Leu Met Asn Arg Thr Gln Thr Asn Leu
   355 360 365
   Thr Glu Gly Pro Pro Asp Lys Glu Cys Ala Val Thr Cys Arg Tyr Asp
   370 375 380
   Lys Asn Thr Asp Val Asn Val Val Thr Gln Ala Arg Asn Arg Pro Thr
   385 390 395 400
   Thr Leu Thr Gly Cys Lys Lys Gly Lys Asn Phe Ser Phe Ala Gly Thr
   405 410 415
   Val Ile Glu Gly Pro Cys Asn Phe Asn Val Ser Val Glu Asp Ile Leu
   420 425 430
   Tyr Gly Asp His Glu Cys Gly Ser Leu Leu Gln Asp Thr Ala Leu Tyr
   435 440 445
   Leu Leu Asp Gly Met Thr Asn Thr Ile Glu Asn Ala Arg Gln Gly Ala
   450 455 460
   Ala Arg Val Thr Ser Trp Leu Gly Arg Gln Leu Ser Thr Ala Gly Lys
   465 470 475 480
   Lys Leu Glu Arg Arg Ser Lys Thr Trp Phe Gly Ala Tyr Ala Leu
   485 490 495
<210> 26
   <211> 494
   <212> PRT
   <213> Artificial Sequence
<220>
   <223> Description of Artificial Sequence: BVDV mutant
<400> 26
   Met Glu Leu Ile Thr Asn Glu Leu Leu Tyr Lys Thr Tyr Lys Gln Lys
    1               5                  10                  15
   Pro Ala Gly Val Glu Glu Pro Val Tyr Asp Gln Ala Gly Asn Pro Leu
               20                  25                  30
   Phe Gly Glu Arg Gly Val Ile His Pro Gln Ser Thr Leu Lys Leu Pro
           35                  40                  45
   His Lys Arg Gly Glu Arg Glu Val Pro Thr Asn Leu Ala Ser Leu Pro
       50                  55                  60
   Lys Arg Gly Asp Cys Arg Ser Gly Asn Ser Lys Gly Pro Val Ser Gly
   65                  70                  75                  80
   Ile Tyr Leu Lys Pro Gly Pro Leu Phe Tyr Gln Asp Tyr Lys Gly Pro
                   85                  90                  95
   Val Tyr His Arg Ala Pro Leu Glu Phe Phe Glu Glu Ala Ser Met Cys
               100                 105                 110
   Glu Thr Thr Lys Arg Ile Gly Arg Val Thr Gly Ser Asp Ser Arg Leu
           115                 120                 125
   Tyr His Ile Tyr Val Cys Ile Asp Gly Cys Ile Ile Val Lys Ser Ala
   130 135 140
   Thr Lys Asp Arg Gln Lys Val Leu Lys Trp Val His Asn Lys Leu Asn
   145 150 155 160
   Cys Pro Leu Trp Val Ser Ser Cys Ser Asp Thr Lys Asp Glu Gly Val
   165 170 175
   Val Arg Lys Lys Gln Gln Lys Pro Asp Arg Leu Glu Lys Gly Arg Met
   180 185 190
   Lys Ile Thr Pro Lys Glu Ser Glu Lys Asp Ser Lys Thr Lys Pro Pro
   195 200 205
   Asp Ala Thr Ile Val Val Asp Gly Val Lys Tyr Gln Val Lys Lys Lys
   210 215 220
   Gly Lys Val Lys Ser Lys Asn Thr Gln Asp Gly Leu Tyr His Asn Lys
   225 230 235 240
   Asn Lys Pro Gln Glu Ser Arg Lys Lys Leu Glu Lys Ala Leu Leu Ala
   245 250 255
   Trp Ala Ile Ile Ala Leu Val Phe Phe Gln Val Thr Met Gly Glu Asn
   260 265 270
   Ile Thr Gln Trp Asn Leu Gln Asp Asn Gly Thr Glu Gly Ile Gln Arg
   275 280 285
   Ala Met Phe Gln Arg Gly Val Asn Arg Ser Leu His Gly Ile Trp Pro
   290 295 300
   Glu Lys Ile Cys Thr Gly Val Pro Ser His Leu Ala Thr Asp Thr Glu
   305 310 315 320
   Leu Lys Ala Ile His Gly Met Met Asp Ala Ser Glu Lys Thr Asn Tyr
   325 330 335
   Thr Cys Cys Arg Leu Gln Arg His Glu Trp Asn Lys Gly Trp Cys Asn
   340 345 350
   Trp Tyr Asn Ile Glu Pro Trp Ile Leu Leu Met Asn Lys Thr Gln Ala
   355 360 365
   Asn Leu Thr Glu Gly Gln Pro Leu Arg Glu Cys Ala Val Thr Cys Arg
   370 375 380
   Tyr Asp Arg Asp Ser Asp Leu Asn Val Val Thr Gln Ala Arg Asp Ser
   385 390 395 400
   Pro Thr Pro Leu Thr Gly Cys Lys Lys Gly Lys Asn Phe Ser Phe Ala
   405 410 415
   Gly Ile Leu Val Gln Gly Pro Cys Asn Phe Glu Ile Ala Val Ser Asp
   420 425 430
   Val Leu Phe Lys Glu His Asp Cys Thr Ser Val Ile Gln Asp Thr Ala
   435 440 445
   His Tyr Leu Val Asp Gly Met Thr Asn Ser Leu Glu Ser Ala Arg Gln
   450 455 460
   Gly Thr Ala Lys Leu Thr Thr Trp Leu Gly Arg Gln Leu Gly Ile Leu
   465                 470                 475                 480
   Gly Lys Lys Leu Glu Asn Lys Ser Lys Thr Trp Phe Gly Ala
                   485                 490
<210> 27
   <211> 495
   <212> PRT
   <213> Artificial Sequence
<220>
   <223> Description of Artificial Sequence: BVDV mutant
<400> 27
   Met Glu Leu Ile Thr Asn Glu Leu Leu Tyr Lys Thr Tyr Lys Gln Lys
   1 5 10 15
   Pro Ala Gly Val Glu Glu Pro Val Tyr Asp Gln Ala Gly Asn Pro Leu
   20 25 30
   Phe Gly Glu Arg Gly Val Ile His Pro Gln Ser Thr Leu Lys Leu Pro
   35 40 45
   His Lys Arg Gly Glu Arg Glu Val Pro Thr Asn Leu Ala Ser Leu Pro
   50 55 60
   Lys Arg Gly Asp Cys Arg Ser Gly Asn Ser Lys Gly Pro Val Ser Gly
   65 70 75 80
   Ile Tyr Leu Lys Pro Gly Pro Leu Phe Tyr Gln Asp Tyr Lys Gly Pro
   85 90 95
   Val Tyr His Arg Ala Pro Leu Glu Phe Phe Glu Glu Ala Ser Met Cys
   100 105 110
   Glu Thr Thr Lys Arg Ile Gly Arg Val Thr Gly Ser Asp Ser Arg Leu
   115 120 125
   Tyr His Ile Tyr Val Cys Ile Asp Gly Cys Ile Ile Val Lys Ser Ala
   130 135 140
   Thr Lys Asp Arg Gln Lys Val Leu Lys Trp Val His Asn Lys Leu Asn
   145 150 155 160
   Cys Pro Leu Trp Val Ser Ser Cys Ser Asp Thr Lys Asp Glu Gly Val
   165 170 175
   Val Arg Lys Lys Gln Gln Lys Pro Asp Arg Leu Glu Lys Gly Arg Met
   180 185 190
   Lys Ile Thr Pro Lys Glu Ser Glu Lys Asp Ser Lys Thr Lys Pro Pro
   195 200 205
   Asp Ala Thr Ile Val Val Asp Gly Val Lys Tyr Gln Val Lys Lys Lys
   210 215 220
   Gly Lys Val Lys Ser Lys Asn Thr Gln Asp Gly Leu Tyr His Asn Lys
   225 230 235 240
   Asn Lys Pro Gln Glu Ser Arg Lys Lys Leu Glu Lys Ala Leu Leu Ala
                       245                 250                 255
   Trp Ala Ile Ile Ala Leu Val Phe Phe Gln Val Thr Met Gly Glu Asn
               260                 265                 270
   Ile Thr Gln Trp Asn Leu Gln Asp Asn Gly Thr Glu Gly Ile Gln Arg
           275                 280                 285
   Ala Met Phe Gln Arg Gly Val Asn Arg Ser Leu His Gly Ile Trp Pro
       290                 295                 300
   Glu Lys Ile Cys Thr Gly Val Pro Ser His Leu Ala Thr Asp Thr Glu
   305                 310                 315                 320
   Leu Lys Ala Ile His Gly Met Met Asp Ala Ser Glu Lys Thr Asn Tyr
                   325                 330                 335
   Thr Cys Cys Arg Leu Gln Arg His Glu Trp Asn Lys His Gly Trp Cys
               340                 345                 350
   Asn Trp Tyr Asn Ile Glu Pro Trp Ile Leu Leu Met Asn Lys Thr Gln
           355                 360                 365
   Ala Asn Leu Thr Glu Gly Gln Pro Leu Arg Glu Cys Ala Val Thr Cys
       370                 375                 380
   Arg Tyr Asp Arg Asp Ser Asp Leu Asn Val Val Thr Gln Ala Arg Asp
   385                 390                 395                 400
   Ser Pro Thr Pro Leu Thr Gly Cys Lys Lys Gly Lys Asn Phe Ser Phe
                   405                 410                 415
   Ala Gly Ile Leu Val Gln Gly Pro Cys Asn Phe Glu Ile Ala Val Ser
               420                 425                 430
   Asp Val Leu Phe Lys Glu His Asp Cys Thr Ser Val Ile Gln Asp Thr
           435                 440                 445
   Ala His Tyr Leu Val Asp Gly Met Thr Asn Ser Leu Glu Ser Ala Arg
       450                 455                 460
   Gln Gly Thr Ala Lys Leu Thr Thr Trp Leu Gly Arg Gln Leu Gly Ile
   465                 470                 475                 480
   Leu Gly Lys Lys Leu Glu Asn Lys Ser Lys Thr Trp Phe Gly Ala
                   485                 490                 495
<210> 28
   <211> 495
   <212> PRT
   <213> Artificial Sequence
<220>
   <223> Description of Artificial Sequence: CSFV Alfort
<400> 28
   Met Glu Leu Asn His Phe Glu Leu Leu Tyr Lys Thr Ser Lys Gln Lys
   1 5 10 15
   Pro Val Gly Val Glu Glu Pro Val Tyr Asp Thr Ala Gly Arg Pro Leu
   20 25 30
   Phe Gly Asn Pro Ser Glu Val His Pro Gln Ser Thr Leu Lys Leu Pro
   35 40 45
   His Asp Arg Gly Arg Gly Asp Ile Arg Thr Thr Leu Arg Asp Leu Pro
   50 55 60
   Arg Lys Gly Asp Cys Arg Ser Gly Asn His Leu Gly Pro Val Ser Gly
   65 70 75 80
   Ile Tyr Ile Lys Pro Gly Pro Val Tyr Tyr Gln Asp Tyr Thr Gly Pro
   85 90 95
   Val Tyr His Arg Ala Pro Leu Glu Phe Phe Asp Glu Ala Gln Phe Cys
   100 105 110
   Glu Val Thr Lys Arg Ile Gly Arg Val Thr Gly Ser Asp Gly Lys Leu
   115 120 125
   Tyr His Ile Tyr Val Cys Val Asp Gly Cys Ile Leu Leu Lys Leu Ala
   130 135 140
   Lys Arg Gly Thr Pro Arg Thr Leu Lys Trp Ile Arg Asn Phe Thr Asn
   145 150 155 160
   Cys Pro Leu Trp Val Thr Ser Cys Ser Asp Asp Gly Ala Ser Gly Ser
   165 170 175
   Lys Asp Lys Lys Pro Asp Arg Met Asn Lys Gly Lys Leu Lys Ile Ala
   180 185 190
   Pro Arg Glu His Glu Lys Asp Ser Lys Thr Lys Pro Pro Asp Ala Thr
   195 200 205
   Ile Val Val Glu Gly Val Lys Tyr Gln Ile Lys Lys Lys Gly Lys Val
   210 215 220
   Lys Gly Lys Asn Thr Gln Asp Gly Leu Tyr His Asn Lys Asn Lys Pro
   225 230 235 240
   Pro Glu Ser Arg Lys Lys Leu Glu Lys Ala Leu Leu Ala Trp Ala Val
   245 250 255
   Ile Thr Ile Leu Leu Tyr Gln Pro Val Ala Ala Glu Asn Ile Thr Gln
   260 265 270
   Trp Asn Leu Ser Asp Asn Gly Thr Asn Gly Ile Gln Arg Ala Met Tyr
   275 280 285
   Leu Arg Gly Val Asn Arg Ser Leu His Gly Ile Trp Pro Glu Lys Ile
   290 295 300
   Cys Lys Gly Val Pro Thr His Leu Ala Thr Asp Thr Glu Leu Lys Glu
   305 310 315 320
   Ile Arg Gly Met Met Asp Ala Ser Glu Arg Thr Asn Tyr Thr Cys Cys
   325 330 335
   Arg Leu Gln Arg His Glu Trp Asn Lys His Gly Trp Cys Asn Trp Tyr
   340 345 350
   Asn Ile Asp Pro Trp Ile Gln Leu Met Asn Arg Thr Gln Thr Asn Leu
   355 360 365
   Thr Glu Gly Pro Pro Asp Lys Glu Cys Ala Val Thr Cys Arg Tyr Asp
       370                 375                 380
   Lys Asn Thr Asp Val Asn Val Val Thr Gln Ala Arg Asn Arg Pro Thr
   385                 390                 395                 400
   Thr Leu Thr Gly Cys Lys Lys Gly Lys Asn Phe Ser Phe Ala Gly Thr
                   405                 410                 415
   Val Ile Glu Gly Pro Cys Asn Phe Asn Val Ser Val Glu Asp Ile Leu
               420                 425                 430
   Tyr Gly Asp His Glu Cys Gly Ser Leu Leu Gln Asp Thr Ala Leu Tyr
           435                 440                 445
   Leu Leu Asp Gly Met Thr Asn Thr Ile Glu Asn Ala Arg Gln Gly Ala
       450                 455                 460
   Ala Arg Val Thr Ser Trp Leu Gly Arg Gln Leu Ser Thr Ala Gly Lys
   465                 470                 475                 480
   Lys Leu Glu Arg Arg Ser Lys Thr Trp Phe Gly Ala Tyr Ala Leu
                   485                 490                 495